(19) Europäisches Patentamt

European Patent Office

Office européen des brevets

(11) **EP 4 585 694 A1**

(12) **EUROPEAN PATENT APPLICATION**
published in accordance with Art. 153(4) EPC

(43) Date of publication:
16.07.2025 Bulletin 2025/29

(21) Application number: 23863283.0

(22) Date of filing: 11.09.2023

(51) International Patent Classification (IPC):
C12P 21/00 (2006.01)    A23L 33/105 (2016.01)
C12N 9/10 (2006.01)    C12N 9/78 (2006.01)
C12P 1/00 (2006.01)

(52) Cooperative Patent Classification (CPC):
A23L 33/105; C12N 9/10; C12N 9/78; C12P 1/00;
C12P 21/00

(86) International application number:
PCT/JP2023/033068

(87) International publication number:
WO 2024/053745 (14.03.2024 Gazette 2024/11)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 09.09.2022 JP 2022143628

(71) Applicant: Amano Enzyme Inc.
Nagoya-shi
Aichi 460-8630 (JP)

(72) Inventor: SAKAI, Kiyota
Kakamigahara-shi, Gifu 509-0109 (JP)

(74) Representative: Müller-Boré & Partner
Patentanwälte PartG mbB
Friedenheimer Brücke 21
80639 München (DE)

(54) **METHOD FOR PRODUCING PROCESSED PLANT PROTEIN-CONTAINING COMPOSITION**

(57) The objective of the present invention is to provide a processing technique that enhances the solubility of a plant protein-containing liquid composition. By treating the plant protein-containing liquid composition with protein deamidase and transglutaminase, the solubility of the plant protein-containing liquid composition or the solubility of a plant protein-containing liquid composition, obtained by re-dispersing a dried product thereof, can be improved.

**EP 4 585 694 A1**

## Description

Technical Field

The present invention relates to a method for producing a processed plant protein-containing composition. More specifically, the present invention relates to a method for enhancing the solubility of a plant protein-containing liquid composition.

Background Art

Drinks rich in nutrients such as proteins have been widely familiar to people because they can easily take nutrients therefrom. On the other hand, in recent years, against the background of an increase in the number of vegetarians, allergic problems, religious reasons, and the like, as an alternative to animal milk typified by cow's milk, drinks made from soybean, oat, almond, and the like rich in plant proteins have been widely spread.

Meanwhile, when a milk protein raw material is intended to be replaced with a plant protein raw material, the milk protein raw material cannot be replaced as it is in many cases because the type and functionality of proteins or the components constituting the aroma and taste are different between the protein raw materials. For this reason, various techniques related to processing of plant protein raw materials have been proposed. For example, PTL 1 describes that the problem of high viscosity is solved by treating an oat suspension with $\alpha$-amylase and $\beta$-amylase, and an oat suspension maintaining proteins and $\beta$-glucans was obtained. PTL 2 describes that dispersion stability and solubility are improved by treating a plant milk with a protein deamidase. On the other hand, a transglutaminase is known as an enzyme that modifies a protein. Transglutaminases are enzymes having an action of crosslinking proteins, and a technique related to processing of dairy products has been proposed. For example, PTL 3 describes that physical properties of yoghurt and cheese can be improved by treating a raw material milk with a transglutaminase.

Citation List

Patent Literature

PTL 1: US Patent No. 6,451,369
PTL 2: WO 2022/071418 A
PTL 3: Japanese Patent Laid-open Publication No. 2002-369653

Summary of Invention

Technical Problem

A plant protein-containing liquid composition is highly useful in terms of nutritional value and storage stability, and can be expected to be used in various applications. Meanwhile, control of the solubility of a plant protein-containing liquid composition (hereinafter, solubility refers to the solubility of a protein in water) has not been sufficiently studied yet.

Therefore, an object of the present invention is to provide a processing technique for improving the solubility of a plant protein-containing liquid composition.

As a result of intensive studies, the present inventor has found that the solubility of a plant protein-containing liquid composition is improved by treating the plant protein-containing liquid composition with a protein deamidase and a transglutaminase. In addition, the present inventor has found that in a plant protein-containing liquid composition obtained by once drying a plant protein-containing liquid composition and then redispersing the dried plant protein-containing liquid composition into a liquid (specifically, water) again, the solubility tends to decrease as compared with a plant protein-containing liquid composition that has not been dried. However, the present inventor has also found that since treating a plant protein-containing liquid composition with a protein deamidase and a transglutaminase is highly effective in improving solubility, the solubility of the plant protein-containing liquid composition by redispersion after drying can also be effectively improved. Furthermore, the present inventor has found that when a plant protein-containing liquid composition derived from a specific plant is selected as a treatment target with a protein deamidase and a transglutaminase, dispersion stability, emulsifiability, emulsion stability, foamability, foam stability, oil retentivity, and/or smoothness are also improved. The present invention has been accomplished by further studies on the basis of these findings.

In summary, the present invention provides aspects of invention as itemized below.

Item 1. A method for producing a processed plant protein-containing composition, including a step of treating a plant protein-containing liquid composition with a protein deamidase and a transglutaminase to obtain a processed plant protein-containing liquid composition.

Item 2. The method for producing a processed plant protein-containing composition according to item 1, further including a step of drying the processed plant protein-containing liquid composition to obtain a processed plant protein-containing dry composition for redispersion in a liquid (water and/or oil).

Item 3. The method for producing a processed plant protein-containing composition according to item 1 or 2, in which the plant protein is a plant-derived protein selected from the group consisting of soybean, pea, mung bean, fava bean, chickpea, lentil, lupine bean, rice, barley, oat, sorghum, rye, corn, potato, hazelnut, cashew nut, almond, coconut, peanut, pistachio, walnut, chia seed, and hemp seed.

Item 4. The method for producing a processed plant protein-containing composition according to any one of items 1 to 3, in which the protein deamidase and the transglutaminase are allowed to act simultaneously.

Item 5. The method for producing a processed plant protein-containing composition according to any one of items 1 to 4, in which an activity ratio of the protein deamidase to the transglutaminase (protein deamidase activity : transglutaminase activity) is 0.05:0.95 to 0.9:0.1.

Item 6. A solubilizer for a plant protein-containing liquid composition, including a protein deamidase and a transglutaminase.

Item 7. The solubilizer according to item 6, in which an activity ratio of the protein deamidase to the transglutaminase (protein deamidase activity : transglutaminase activity) is 0.05:0.95 to 0.9:0.1.

Item 8. The solubilizer according to item 6, which is used for improving dispersion stability of an oat-derived plant protein-containing liquid composition.

Item 9. The solubilizer according to item 8, in which an activity ratio of the protein deamidase to the transglutaminase (protein deamidase activity : transglutaminase activity) is 0.05:0.95 to 0.9:0.1.

Item 10. The solubilizer according to item 6 or 7, which is used for improving emulsifiability of a plant protein-containing liquid composition derived from a plant selected from the group consisting of soybean, pea, mung bean, fava bean, chickpea, lentil, rice, corn, cashew nut, almond, coconut, pistachio, walnut, and chia seed.

Item 11. The solubilizer according to item 6 or 7, which is used for improving emulsion stability of a plant protein-containing liquid composition derived from a plant selected from the group consisting of soybean, pea, fava bean, lentil, rice, barley, sorghum, potato, hazelnut, almond, coconut, peanut, pistachio, and hemp seed.

Item 12. The solubilizer according to item 6 or 7, which is used for improving foamability of a plant protein-containing liquid composition derived from a plant selected from the group consisting of pea, mung bean, chickpea, and almond.

Item 13. The solubilizer according to item 6 or 7, which is used for improving foam stability of a plant protein-containing liquid composition derived from a plant selected from the group consisting of soybean, fava bean, rice, and potato.

Item 14. The solubilizer according to item 6 or 7, which is used for improving oil retentivity of a plant protein-containing liquid composition derived from a plant selected from the group consisting of fava bean, chickpea, lentil, rice, potato, cashew nut, peanut, pistachio, walnut, chia seed, and hemp seed.

Item 15. The solubilizer according to item 6 or 7, which is used for improving smoothness of a plant protein-containing liquid composition derived from a plant selected from the group consisting of soybean, pea, mung bean, lupine bean, rice, and almond.

Item 16. A plant protein-containing food or drink including a processed plant protein-containing composition obtained by the production method according to any one of items 1 to 5.

Item 17.

(a) A method for improving solubility of a plant protein-containing liquid composition, including a step of treating a plant protein-containing liquid composition with a protein deamidase and a transglutaminase to obtain a processed plant protein-containing liquid composition.

(b) The method according to the above (a), in which the method is a method for solubilization and dispersion stability improvement, and the plant protein-containing liquid composition is an oat-derived plant protein-containing liquid composition.

(c) The method according to the above (a), in which the method is a method for solubilization and emulsifiability improvement, and the plant protein-containing liquid composition is a plant protein-containing liquid composition derived from a plant selected from the group consisting of soybean, pea, mung bean, fava bean, chickpea, lentil, rice, corn, cashew nut, almond, coconut, pistachio, walnut, and chia seed.

(d) The method according to the above (a), in which the method is a method for solubilization and emulsion stability improvement, and the plant protein-containing liquid composition is a plant protein-containing liquid composition derived from a plant selected from the group consisting of soybean, pea, fava bean, lentil, rice, barley, sorghum, potato, hazelnut, almond, coconut, peanut, pistachio, and hemp seed.

(e) The method according to the above (a), in which the method is a method for solubilization and foamability improvement, and the plant protein-containing liquid composition is a plant protein-containing liquid composition derived from a plant selected from the group consisting of pea, mung bean, chickpea, and almond.

(f) The method according to the above (a), in which the method is a method for solubilization and foam stability improvement, and the plant protein-containing liquid composition is a plant protein-containing liquid composition derived from a plant selected from the group consisting of soybean, fava bean, rice, and potato.

(g) The method according to the above (a), in which the method is a method for solubilization and oil retentivity improvement, and the plant protein-containing liquid composition is a plant protein-containing liquid composition derived from a plant selected from the group consisting of fava bean, chickpea, lentil, rice, potato, cashew nut, peanut, pistachio, walnut, chia seed, and hemp seed.

(h) The method according to the above (a), in which the method is a method for solubilization and smoothness improvement, and the plant protein-containing liquid composition is a plant protein-containing liquid composition derived from a plant selected from the group consisting of soybean, pea, mung bean, lupine bean, rice, and almond.

Item 18.
Use of a protein deamidase and a transglutaminase as

(a) solubilization of a plant protein-containing liquid composition;

(b) solubilization and dispersion stability improvement of an oat-derived plant protein-containing liquid composition;

(c) solubilization and emulsifiability improvement of a plant protein-containing liquid composition derived from a plant selected from the group consisting of soybean, pea, mung bean, fava bean, chickpea, lentil, rice, corn, cashew nut, almond, coconut, pistachio, walnut, and chia seed;

(d) solubilization and emulsion stability improvement of a plant protein-containing liquid composition derived from a plant selected from the group consisting of soybean, pea, fava bean, lentil, rice, barley, sorghum, potato, hazelnut, almond, coconut, peanut, pistachio, and hemp seed;

(e) solubilization and foamability improvement of a plant protein-containing liquid composition derived from a plant selected from the group consisting of pea, mung bean, chickpea, and almond;

(f) solubilization and foam stability improvement of a plant protein-containing liquid composition derived from a plant selected from the group consisting of soybean, fava bean, rice, and potato;

(g) solubilization and oil retentivity improvement of a plant protein-containing liquid composition derived from a plant selected from the group consisting of fava bean, chickpea, lentil, rice, potato, cashew nut, peanut, pistachio, walnut, chia seed, and hemp seed; or

(h) solubilization and smoothness improvement of a plant protein-containing liquid composition derived from a plant selected from the group consisting of soybean, pea, mung bean, lupine bean, rice, and almond.

Item 19.
Application of a protein deamidase and a transglutaminase as

(a) a solubilizer for a plant protein-containing liquid composition;

(b) an agent for solubilization and dispersion stability improvement of an oat-derived plant protein-containing liquid composition;

(c) an agent for solubilization and emulsifiability improvement of a plant protein-containing liquid composition derived from a plant selected from the group consisting of soybean, pea, mung bean, fava bean, chickpea, lentil, rice, corn, cashew nut, almond, coconut, pistachio, walnut, and chia seed;

(d) an agent for solubilization and emulsion stability improvement of a plant protein-containing liquid composition derived from a plant selected from the group consisting of soybean, pea, fava bean, lentil, rice, barley, sorghum, potato, hazelnut, almond, coconut, peanut, pistachio, and hemp seed;

(e) an agent for solubilization and foamability improvement of a plant protein-containing liquid composition derived from a plant selected from the group consisting of pea, mung bean, chickpea, and almond;

(f) an agent for solubilization and foam stability improvement of a plant protein-containing liquid composition derived from a plant selected from the group consisting of soybean, fava bean, rice, and potato;

(g) an agent for solubilization and oil retentivity improvement of a plant protein-containing liquid composition derived from a plant selected from the group consisting of fava bean, chickpea, lentil, rice, potato, cashew nut, peanut, pistachio, walnut, chia seed, and hemp seed; or

(h) an agent for solubilization and smoothness improvement of a plant protein-containing liquid composition derived from a plant selected from the group consisting of soybean, pea, mung bean, lupine bean, rice, and almond.

Advantageous Effects of Invention

According to the present invention, there is provided a processing technique for improving the solubility of a plant protein-containing liquid composition. **In** addition, when an oat-derived plant protein-containing liquid composition is selected as the plant protein-containing liquid composition, a processing technique for improving dispersion stability as well is provided; when a plant protein-containing liquid composition derived from a plant selected from the group consisting of soybean, pea, mung bean, fava bean, chickpea, lentil, rice, corn, cashew nut, almond, coconut, pistachio, walnut, and chia seed is selected as the plant protein-containing liquid composition, a processing technique for improving emulsifiability as well is provided; when a plant protein-containing liquid composition derived from a plant selected from the group consisting of soybean, pea, fava bean, lentil, rice, barley, sorghum, potato, hazelnut, almond, coconut, peanut, pistachio, and hemp seed is selected as the plant protein-containing liquid composition, a processing technique for improving emulsion stability as well is provided; when a plant protein-containing liquid composition derived from a plant selected from the group consisting of pea, mung bean, chickpea, and almond is selected as the plant protein-containing liquid composition, a processing technique for improving foamability as well is provided; when a plant protein-containing liquid composition derived from a plant selected from the group consisting of soybean, fava bean, rice, and potato is selected as the plant protein-containing liquid composition, a processing technique for improving foam stability as well is provided; when a plant protein-containing liquid composition derived from a plant selected from the group consisting of fava bean, chickpea, lentil, rice, potato, cashew nut, peanut, pistachio, walnut, chia seed, and hemp seed is selected as the plant protein-containing liquid composition, a processing technique for improving oil retentivity as well is provided; when a plant protein-containing liquid composition derived from a plant selected from the group consisting of soybean, pea, mung bean, lupine bean, rice, and almond is selected as the plant protein-containing liquid composition, a processing technique for improving smoothness as well is provided.

Description of Embodiments

1. Method for producing processed plant protein-containing composition

The method for producing a processed plant protein-containing composition of the present invention is characterized by including a step (enzyme treatment step) of treating a plant protein-containing liquid composition with a protein deamidase and a transglutaminase. The solubility of the protein in the plant protein-containing liquid composition can be improved by the enzyme treatment step, and therefore, according to the production method of the present invention, a plant protein-containing composition processed for improving solubility is obtained. In addition, when a plant protein-containing liquid composition derived from a specific plant is selected as an enzyme treatment target, it is possible to improve dispersion stability, emulsifiability, emulsion stability, foamability, foam stability, oil retentivity, and/or smoothness in addition to solubility. Therefore, according to the production method of the present invention, a plant protein-containing composition processed for improving dispersion stability, emulsifiability, emulsion stability, foamability, foam stability, oil retentivity, and/or smoothness in addition to solubility is obtained. Hereinafter, the method for producing a processed plant protein-containing composition of the present invention will be described in detail.

1-1. Plant protein-containing liquid composition

The plant protein-containing liquid composition to be used in the present invention is not particularly limited as long as it is a liquid containing a plant protein in water. Examples of the plant protein-containing liquid composition include (i) liquids obtained by dispersing a dry powder of a food material containing a plant protein in water, (ii) liquids obtained by crushing and dispersing a food material containing a plant protein in water and, as necessary, removing an insoluble matter derived from a food material skin and the like by any means such as centrifugation, filtration, a filter bag, or a sieve, (iii) liquids in which the plant protein content is increased by, for example, removing a component other than the plant protein from a liquid of (i) or (ii) described above, and (iv) liquids obtained by mixing a dry powder prepared from any liquid of (i) to (iii) described above with water. Preferable examples of the plant protein-containing liquid composition include plant milk.
In the following description, the expression "content of a plant protein material" refers to the dry weight of the components derived from plants including plant proteins in the plant protein-containing liquid composition.
Examples of the plant protein include, but are not particularly limited to, proteins (plant-derived proteins, i.e., natural proteins) contained in beans such as soybean, pea, mung bean, fava bean, chickpea, lentil, black bean, lupine bean, and kidney bean, cereals such as rice, wheat, barley, oat, sorghum, rye, buckwheat, Japanese millet, foxtail millet, teff, corn, and potato, nuts such as hazelnut, cashew nut, almond, coconut, peanut, pistachio, walnut, pecan nut, macadamia nut, Brazil nut, pilinut, chestnut, sesame, and pine nut, and seeds such as chia seed, hemp seed (industrial hemp free of tetrahydrocannabinol (THC)), quinoa, amaranthus, canary seed, and linseed; the above-described proteins chemically partially decomposed by an acid, an alkali, or the like; proteins chemically modified with various reagents, and synthetic peptides.

In the present invention, the plant proteins described above may be used singly or two or more of them may be used in combination.

Among the above plant proteins, from the viewpoint of further improving the solubility, preferred are proteins derived from at least any among soybean, pea, mung bean, fava bean, chickpea, lentil, lupine bean, rice, barley, oat, sorghum, rye, corn, potato, hazelnut, cashew nut, almond, coconut, peanut, pistachio, walnut, chia seed, and hemp seed, more preferred are proteins derived from at least any among soybean, fava bean, lupine bean, rice, rye, corn, potato, coconut, peanut, and hemp seed, further preferred are proteins derived from at least any among lupine bean, corn, potato, and peanut, further preferred are proteins derived from at least any among lupine bean, potato, and peanut, and particularly preferred is a protein derived from lupine bean.

Among the above plant proteins, oat is preferred from the viewpoint of further improving dispersion stability.

Among the above plant proteins, from the viewpoint of further improving the emulsifiability, preferred are proteins derived from at least any among soybean, pea, mung bean, fava bean, chickpea, lentil, rice, corn, cashew nut, almond, coconut, pistachio, walnut, and chia seed, more preferred are proteins derived from at least any among corn, coconut, and walnut, still more preferred are proteins derived from at least any of corn and walnut, and particularly preferred is a protein derived from corn.

Among the above plant proteins, from the viewpoint of further improving the emulsion stability, preferred are proteins derived from at least any among soybean, pea, fava bean, lentil, rice, barley, sorghum, potato, hazelnut, almond, coconut, peanut, pistachio, and hemp seed, more preferred are proteins derived from at least any among soybean, pea, fava bean, lentil, rice, barley, sorghum, potato, coconut, and pistachio, still more preferred are proteins derived from at least any among soybean, pea, fava bean, lentil, rice, sorghum, and pistachio, and particularly preferred are proteins derived from at least any among lentil, rice, and pistachio.

Among the above plant proteins, from the viewpoint of further improving the foamability, preferred are proteins derived from at least any among pea, mung bean, chickpea, and almond, more preferred are proteins derived from at least any of chickpea and almond, and particularly preferred is a protein derived from almond.

Among the above plant proteins, from the viewpoint of further improving the foam stability, preferred are proteins derived from at least any among soybean, fava bean, rice, and potato, more preferred are proteins derived from at least any among soybean, fava bean, and potato, and particularly preferred is a protein derived from soybean.

Among the above plant proteins, from the viewpoint of further improving the oil retentivity, preferred are proteins derived from at least any among fava bean, chickpea, lentil, rice, potato, cashew nut, peanut, pistachio, walnut, chia seed, and hemp seed, more preferred are proteins derived from at least any among lentil, potato, cashew nut, pistachio, chia seed, and hemp seed, still more preferred are proteins derived from at least any among potato, cashew nut, pistachio, and chia seed, and particularly preferred are proteins derived from at least any of potato and chia seed.

Among the above plant proteins, from the viewpoint of further improving the smoothness, preferred are proteins derived from at least any among soybean, pea, mung bean, lupine bean, rice, and almond, and more preferred are proteins derived from at least any among soybean, pea, mung bean, lupine bean, and rice.

The content of the plant protein in the plant protein-containing liquid composition is not particularly limited, and, for example, is 0.01 to 50% by weight, 0.05 to 40% by weight, or 0.1 to 20% by weight, preferably 0.25 to 10% by weight, or 0.5 to 5.0% by weight, and more preferably 1.0 to 2.0% by weight, or 1.2 to 1.8% by weight.

The content of the plant protein material in the plant protein-containing liquid composition is not particularly limited, and, for example, is 0.05 to 50% by weight, 0.1 to 40% by weight, or 0.5 to 30% by weight, preferably 1 to 25% by weight, or 3 to 20% by weight, and more preferably 5 to 15% by weight, or 10 to 13% by weight.

When the plant protein contained in the plant protein-containing liquid composition is a protein of a plant rich in starch (preferably cereals), such a plant protein-containing liquid composition is preferably one pretreated with an amylase. Examples of the amylase typically include $\alpha$-amylase and $\beta$-amylase.

The $\alpha$-amylase is not particularly limited, and examples thereof include $\alpha$ amylases derived from the genus Aspergillus (e.g., Aspergillus oryzae and Aspergillus niger); and the genus Bacillus (e.g., Bacillus amyloliquefaciens, Bacillus subtilis, and Bacillus licheniformis). An $\alpha$-amylase derived from the genus Bacillus is preferable, and an $\alpha$-amylase derived from Bacillus amyloliquefaciens species is more preferable.

The amount of $\alpha$-amylase used is, for example, 0.5 to 100 U, 1 to 50 U, 2 to 30 U, or 5 to 20 U, preferably 8 to 12 U per 1 g of starch contained in the plant protein-containing liquid composition.

For the activity of $\alpha$-amylase, the amount of the enzyme that reduces coloring of potato starch due to iodine by 10% per minute is defined as 1 unit (1 U).

The $\beta$-amylase is not particularly limited, and examples thereof include a $\beta$-amylase derived from a plant (wheat or soybean) and a $\beta$-amylase derived from the genus Bacillus. A $\beta$-amylase derived from the genus Bacillus is preferable, and a $\beta$-amylase derived from Bacillus flexus species is more preferable.

The amount of $\beta$-amylase used is, for example, 0.001 to 5 U, 0.01 to 1 U, 0.02 to 0.50 U, or 0.03 to 0.25 U, preferably 0.05 to 0.15 U per 1 g of starch contained in the plant protein-containing liquid composition.

For the activity of β-amylase, the amount of the enzyme that leads to an increase in reducing power corresponding to 1 mg of glucose per minute is defined as 1 unit (1 U).

The pH (at 25°C) of the plant protein-containing liquid composition may be appropriately determined according to the optimal pH of the protein deamidase and the transglutaminase, and the like, and is, for example, 4.0 to 9.0, preferably 5.0 to 8.5, more preferably 5.5 to 8.0, and further preferably 5.8 to 7.0.

1-2. Protein deamidase

The protein deamidase to be used in the present invention is an enzyme that exhibits an action of decomposing an amide group-containing side chain of a protein without cleaving peptide bonds and without crosslinking the protein, and the type, the origin, and the like of the enzyme are not particularly limited.

Examples of the protein deamidase include enzymes that deamidate a glutamine residue in a protein and convert the residue into a glutamic acid residue (e.g., protein-glutaminases) and enzymes that deamidate an asparagine residue in a protein and convert the residue into an aspartic acid residue (e.g., protein-asparaginases). Among these protein deamidases, protein-glutaminases are preferred.

More specific examples of the protein deamidase include protein deamidases derived from the genera Chryseobacterium, Flavobacterium, Empedobacter, Sphingobacterium, Aureobacterium, Myroides, Luteimicrobium, Agromyces, Microbacterium, and Leifsonia. These protein deamidases are known, and for example, JP 2000-50887 A, JP 2001-218590 A, WO 2006/075772 A1, and WO 2015/133590 can be referred to. These protein deamidase may be used singly or two or more thereof may be used in combination.

Among these protein deamidases, from the viewpoint of further improving the effect of improving the solubility or additionally from the viewpoint of also improving the dispersion stability, the emulsifiability, the emulsion stability, the foamability, the foam stability, the oil retentivity, and/or the smoothness, protein deamidases derived from the genus Chryseobacterium are preferable, protein-glutaminases derived from the genus Chryseobacterium are more preferable, protein-glutaminases derived from Chryseobacterium proteolyticum species are still more preferable, and a protein-glutaminase derived from Chryseobacterium proteolyticum strain 9670 is further preferable.

The protein deamidase can be prepared from a culture solution of a microorganism as an origin of the above protein deamidase. A specific preparation method may be a method of collecting a protein deamidase from a culture solution or a bacterial cell of the above microorganism. For example, in the case of using a microorganism that secrets a protein deamidase, the enzyme can be separated and/or purified after bacterial cells are collected from the culture solution by filtration, centrifugation, or the like in advance, as necessary. In the case of using a microorganism that does not secret a protein deamidase, after bacterial cells are collected from the culture solution in advance, as necessary, the bacterial cells are disrupted by pressurization treatment, ultrasonic treatment, or the like to expose the enzyme, and then the enzyme can be separated and/or purified. As an enzyme separation and/or purification method, a known protein separation and/or purification method can be used without particular limitation, and examples thereof include a centrifugal separation method, a UF concentration method, a salting-out method, and various chromatography methods using an ion exchange resin. The separated and/or purified enzyme can be pulverized by a drying method such as freeze-drying or reduced-pressure drying, and can also be pulverized using an appropriate excipient and/or drying aid in the drying method. The separated and/or purified enzyme can also be liquefied by adding an appropriate additive and subjecting it to filtration sterilization.

As the protein deamidase, a commercially available product can also be used, and examples of a preferable commercially available product include a protein glutaminase (derived from Chryseobacterium proteolyticum) manufactured by Amano Enzyme Inc.

The amount of the protein deamidase used is not particularly limited, and is, for example, 0.1 U or more per 1 g of the plant protein. From the viewpoint of further improving the effect of improving the solubility, or additionally from the viewpoint of improving the dispersion stability, the emulsifiability, the emulsion stability, the foamability, the foam stability, the oil retentivity and/or the smoothness, the amount of the protein deamidase used per 1 g of the plant protein is preferably 0.5 U or more, or 1 U or more, more preferably 3 U or more, or 4 U or more, still more preferably 10 U or more, further preferably 15 U or more, 30 U or more, 50 U or more, 70 U or more, 90 U or more, 130 U or more, or 170 U or more.

The range of the amount of the protein deamidase used per 1 g of the plant protein is not particularly limited on the upper limit thereof, and examples thereof include 4000 U or less, 3000 U or less, 2000 U or less, 1000 U or less, 500 U or less, 400 U or less, 300 U or less, 200 U or less, 150 U or less, 100 U or less, 90 U or less, 80 U or less, 60 U or less, 50 U or less, 40 U or less, 30 U or less, or 25 U or less.

The amount of the protein deamidase used per 1 g (in terms of dry weight) of the plant protein material is, for example, 0.01 U or more. From the viewpoint of further improving the effect of improving the solubility, or additionally from the viewpoint of improving the dispersion stability, the emulsifiability, the emulsion stability, the foamability, the foam stability, the oil retentivity and/or the smoothness, the amount of the protein deamidase used per 1 g (in terms of dry weight) of the plant protein material is preferably 0.05 U or more, or 0.1 U or more, more preferably 0.5 U or more, still more preferably 0.8 U or more, further preferably 1 U or more, and still further preferably 2 U or more. The range of the amount of the protein deamidase used per 1 g (in terms of dry weight) of the plant protein material is not particularly limited on the upper limit thereof, and examples thereof include 400 U or less, 300 U or less, 200 U or less, 100 U or less, 50 U or less, 40 U or less, 30 U or less, 25 U or less, 20 U or less, 15 U or less, 10 U or less, 9 U or less, or 8 U or less.

For the activity of the protein deamidase, the amount of enzyme liberating 1 μmol of ammonia per minute using benzyloxycarbonyl-L-glutaminylglycine (Z-Gln-Gly) as a substrate is defined as 1 unit (1 U).

1-3. Transglutaminase

The transglutaminase to be used in the present invention is any enzyme having transglutaminase activity (EC2.3.2.13). As the transglutaminase, both a calcium-dependent transglutaminase, which requires calcium for active expression, and a calcium-independent transglutaminase, which does not require calcium for active expression, are mentioned. The transglutaminase is an enzyme having an activity of crosslinking a protein by catalyzing an acyl rearrangement reaction between a γ-carboxamide group of a glutamine residue and an ε-amino group of a lysine residue in the protein.

Examples of the transglutaminase to be used in the present invention are not particularly limited. Examples of the transglutaminase specifically include transglutaminases derived from microorganisms, specifically, transglutaminases derived from the genus Streptomyces such as Streptomyces mobaraensis, Streptomyces ladakanum, Streptomyces cinnamoneus, Streptomyces griseocarneus, Streptomyces lavendulae, or Streptomyces lydicus (see, for example, JPS 64-27471 A), transglutaminases derived from the genus Kutzneria such as Kutzneria albida (see, for example, JP 2020-195397 A), and transglutaminases derived from the genus Longimycelium such as Longimycelium tulufanense; transglutaminases derived from mammals (see, for example, JPH 01-50382 B); transglutaminases derived from fish (see, for example, JPH 06-113844 A); recombinant transglutaminases obtained using the recombinant DNA technology (see, for example, JPH 5-199883 A and JP 2004-97099 A); and transglutaminases having a structure in which a prosequence peptide of a transglutaminase is bound to a mature transglutaminase (see, for example, WO 2009/101762 A). These transglutaminases may be used singly, or two or more of them may be used in combination.

Among these transglutaminases, from the viewpoint of further enhancing the effect of improving the solubility, or additionally from the viewpoint of improving the dispersion stability, the emulsifiability, the emulsion stability, the foamability, the foam stability, the oil retentivity, and/or the smoothness, transglutaminases derived from microorganisms are preferable, transglutaminases derived from the genus Streptomyces are more preferable, and transglutaminases derived from Streptomyces mobaraensis species are still more preferable.

The amount of the transglutaminase used is not particularly limited, and is, for example, 0.1 U or more per 1 g of the plant protein. From the viewpoint of further improving the effect of improving the solubility, or additionally from the viewpoint of improving the dispersion stability, the emulsifiability, the emulsion stability, the foamability, the foam stability, the oil retentivity and/or the smoothness, the amount of the transglutaminase used per 1 g of the plant protein is preferably 0.3 U or more, or 0.5 U or more, more preferably 1 U or more, 2.5 U or more, or 5 U or more, still more preferably 8 U or more, and further preferably 10 U or more, 20 U or more, 30 U or more, 40 U or more, 50 U or more, 60 U or more, 70 U or more, or 80 U or more.

The range of the amount of the transglutaminase used per 1 g of the plant protein is not particularly limited on the upper limit thereof, and examples thereof include 4000 U or less, 3000 U or less, 2000 U or less, 1000 U or less, 500 U or less, 400 U or less, 300 U or less, 200 U or less, 150 U or less, 100 U or less, 90 U or less, 80 U or less, 60 U or less, 50 U or less, 40 U or less, 30 U or less, or 25 U or less.

The amount of the transglutaminase used per 1 g (in terms of dry weight) of the plant protein material is, for example, 0.01 U or more. From the viewpoint of further improving the effect of improving the solubility, or additionally from the viewpoint of improving the dispersion stability, the emulsifiability, the emulsion stability, the foamability, the foam stability, the oil retentivity and/or the smoothness, the amount of the transglutaminase used per 1 g (in terms of dry weight) of the plant protein material is preferably 0.05 U or more, or 0.1 U or more, more preferably 0.5 U or more, still more preferably 0.8 U or more, further preferably 1 U or more, and still further preferably 2.5 U or more, or 3 U or more. The range of the amount of the transglutaminase used per 1 g of the plant protein material (in terms of dry weight) is not particularly limited on the upper limit thereof, and examples thereof include 400 U or less, 300 U or less, 200 U or less, 100 U or less, 50 U or less, 40 U or less, 30 U or less, 20 U or less, 15 U or less, 10 U or less, 9 U or less, or 8 U or less.

The use ratio of the protein deamidase to the transglutaminase is determined on the basis of the above use amount for each enzyme, and examples thereof include a ratio at which the activity ratio of the protein deamidase activity to the transglutaminase activity (protein deamidase activity : transglutaminase activity) is 0.01:0.99 to 0.99:0.01. From the viewpoint of further improving the effect of improving the solubility, or additionally from the viewpoint of improving the dispersion stability, the emulsifiability, the emulsion stability, the foamability, the foam stability, the oil retentivity and/or the smoothness, preferred is a ratio at which the activity ratio is 0.05:0.95 to 0.9:0.1. From the viewpoint of further improving the effect of improving the solubility, or additionally from the viewpoint of improving the emulsifiability, the emulsion stability, the foamability, the foam stability, the oil retentivity and/or the smoothness, preferred is a ratio at which the activity ratio is 0.1:0.9 to 0.9:0.1, more preferably 0.3:0.7 to 0.9:0.1, still more preferably 0.45:0.55 to 0.8:0.2, still more preferably 0.6:0.4 to 0.75:0.25, and particularly preferably 0.65:0.35 to 0.7:0.3. From the viewpoint of further improving the effect of improving the dispersion stability, preferred is a ratio at which the activity ratio is 0.05:0.95 to 0.75:0.25, more preferably 0.08:0.92 to 0.7:0.3 or 0.08:0.92 to 0.4:0.6, still more preferably 0.08:0.92 to 0.25:0.75, and further preferably 0.08:0.92 to 0.15:0.85.

For the activity of the transglutaminase, the enzyme activity of producing 1 μmol of hydroxamic acid per minute in the case of using benzyloxycarbonyl-L-glutaminylglycine and hydroxylamine as substrates is defined as 1 unit (U).

1-4. Reaction conditions, etc.

In an enzyme treatment step, a reaction for improving the solubility of the protein in the plant protein-containing liquid composition or a reaction for improving the dispersion stability, the emulsifiability, the emulsion stability, the foamability, the foam stability, the oil retentivity and/or the smoothness in addition to the solubility is advanced by this treatment.

The order of actions of the protein deamidase and the transglutaminase is not particularly limited, and the enzymes may be sequentially made to act in any order, or both the enzymes may be simultaneously made to act. Preferably, both the enzymes may be made to act simultaneously.

The conditions (temperature, pH, time, etc.) of the enzyme treatment step are appropriately selected according to the properties of the enzymes to be used and the plant protein-containing liquid composition, and also according to the intended effect of improving the solubility, or the degree of the effect of improving the solubility and the effect of improving the dispersion stability, the emulsifiability, the emulsion stability, the foamability, the foam stability, the oil retentivity, and/or the smoothness.

The temperature at which the enzyme treatment step is performed is not particularly limited, and may be appropriately determined by those skilled in the art according to, for example, the optimum temperature of the enzymes to be used and/or the thermal characteristics of the plant protein-containing liquid composition, and is, for example, 30°C to 90°C, preferably 40°C to 80°C, more preferably 48 to 70°C, and still more preferably 50°C to 65°C.

The pH of the reaction system in which the enzyme treatment step is performed is not particularly limited, and may be appropriately determined by those skilled in the art according to, for example, the optimum pH of the enzymes to be used and/or the pH characteristics of the plant protein-containing liquid composition, and the pH at 25°C is, for example, 2 to 13, preferably 5 to 11, and more preferably 5 to 10, 6 to 10, 7 to 9, or 6 to 8.

The time for which the enzyme treatment step is performed is, for example, 0.1 hours to 96 hours, preferably 0.25 hours to 72 hours, and more preferably 0.5 hours to 30 hours.

The plant protein-containing liquid composition after completion of the enzyme treatment is subjected to an enzyme deactivation step as necessary, cooled, and further subjected to a post-treatment step such as filtration as necessary to afford a processed plant protein-containing liquid composition.

Furthermore, the resulting processed plant protein-containing liquid composition may also be prepared as a solid processed plant protein-containing composition (processed plant protein-containing dry composition) through a drying step.

In general, when the plant protein-containing liquid composition is once dried and then redispersed in a liquid (specifically, water) again, the solubility of the plant protein-containing liquid composition formed by such redispersion tends to decrease as compared with the plant protein-containing liquid composition that has not been dried. However, a high effect of improving the solubility is attained by treatment with a protein deamidase and a transglutaminase, and therefore even in the case where the resulting processed plant protein-containing liquid composition has once been dried, the solubility of the plant protein-containing liquid composition by redispersion can be effectively improved. **In** addition, generally, when the plant protein-containing liquid composition is once dried and then redispersed in a liquid (specifically, water and/or oil) again, the plant protein-containing liquid composition resulting from such redispersion tends to deteriorate also in the dispersion stability, the emulsifiability, the emulsion stability, the foamability, the foam stability, the oil retentivity, and/or the smoothness as compared with the plant protein-containing liquid composition that has not been dried. However, a high effect of improving the dispersion stability, the emulsifiability, the emulsion stability, the foamability, the foam stability, the oil retentivity, and/or the smoothness is attained by treatment with a protein deamidase and a transglutaminase, and therefore even in the case where the resulting processed plant protein-containing liquid composition has once been dried, the dispersion stability, the emulsifiability, the emulsion stability, the foamability, the foam stability, the oil retentivity, and/or the smoothness of the plant protein-containing liquid composition by redispersion can be effectively improved.

The method of drying is not particularly limited, and examples of the method include freeze-drying, vacuum drying, and spray drying. Examples of the shape of the solid processed plant protein-containing composition (processed plant protein-containing dry composition) include powder, fine particles, and granules.

2. Solubilizer for plant protein-containing liquid composition

The combination of a protein deamidase and a transglutaminase can improve the solubility of the protein of a plant protein-containing liquid composition. Therefore, the present invention also provides a solubilizer for a plant protein-containing liquid composition including a protein deamidase and a transglutaminase. The solubilizer of a plant protein-containing liquid composition may be in the form of an enzyme preparation including a protein deamidase and a transglutaminase.

From the viewpoint of further improving the solubility, preferred as the plant protein are proteins derived from at least any among soybean, pea, mung bean, fava bean, chickpea, lentil, lupine bean, rice, barley, oat, sorghum, rye, corn, potato, hazelnut, cashew nut, almond, coconut, peanut, pistachio, walnut, chia seed, and hemp seed, more preferred are proteins derived from at least any among soybean, fava bean, lupine bean, rice, rye, corn, potato, coconut, peanut, and hemp seed, further preferred are proteins derived from at least any among lupine bean, corn, potato, and peanut, further preferred are proteins derived from at least any among lupine bean, potato, and peanut, and particularly preferred is a protein derived from lupine bean.

In the above solubilizer, the type, the use amount, etc. of the component to be used are as described in the section "1. Method for producing processed plant protein-containing composition".

The solubilizer of the plant protein-containing liquid composition may be, in a preferred form, one to be used for improving the dispersion stability in addition to solubilization (hereinafter, referred to as "agent for solubilization and dispersion stability improvement"), one to be used for improving the emulsifiability in addition to solubilization (hereinafter, referred to as "agent for solubilization and emulsifiability improvement"), one to be used for improving the emulsion stability in addition to solubilization (hereinafter, referred to as "agent for solubilization and emulsion stability improvement"), one to be used for improving the foamability in addition to solubilization (hereinafter, referred to as "agent for solubilization and foamability improvement"), one to be used for improving the foam stability in addition to solubilization (hereinafter, referred to as "agent for solubilization and foam stability improvement"), one to be used for improving the oil retentivity in addition to solubilization (hereinafter, referred to as "agent for solubilization and oil retentivity improvement"), or one to be used for improving the smoothness in addition to solubilization (hereinafter, referred to as "agent for solubilization and smoothness improvement").

2-1. Agent for solubilization and dispersion stability improvement

In a preferred form, the combination of the protein deamidase and the transglutaminase can improve the dispersion stability in addition to the solubilization of the plant protein-containing liquid composition. Therefore, the present invention also provides an agent for solubilization and dispersion stability improvement of a plant protein-containing liquid composition, including a protein deamidase and a transglutaminase.

From the viewpoint of further improving the dispersion stability, the plant protein is preferably an oat-derived protein.

In the agent for solubilization and dispersion stability improvement described above, the type, the use amount, etc. of the component to be used are as described in the section "1. Method for producing processed plant protein-containing composition".

2-2. Agent for solubilization and emulsifiability improvement

The combination of the protein deamidase and the transglutaminase can improve the emulsifiability in addition to the solubilization of a plant protein-containing liquid composition derived from a plant selected from the group consisting of soybean, pea, mung bean, fava bean, chickpea, lentil, rice, corn, cashew nut, almond, coconut, pistachio, walnut, and chia seed. Accordingly, the present invention also provides an agent for solubilization and emulsifiability improvement of a plant protein-containing liquid compositions derived from these specific plants, including a protein deamidase and a transglutaminase.

From the viewpoint of further improving the emulsifiability, preferred as the plant protein are proteins derived from at least any among corn, coconut, and walnut, more preferred are proteins derived from at least any of corn and walnut, and particularly preferred is a protein derived from corn.

In the agent for solubilization and emulsifiability improvement described above, the type, the use amount, etc. of the component to be used are as described in the section "1. Method for producing processed plant protein-containing composition".

2-3. Agent for solubilization and emulsion stability improvement

The combination of the protein deamidase and the transglutaminase can improve the emulsion stability in addition to the solubilization of a plant protein-containing liquid composition derived from a plant selected from the group consisting of soybean, pea, fava bean, lentil, rice, barley, sorghum, potato, hazelnut, almond, coconut, peanut, pistachio, and hemp seed. Accordingly, the present invention also provides an agent for solubilization and emulsion stability improvement of a plant protein-containing liquid compositions derived from these specific plants, including a protein deamidase and a transglutaminase.

From the viewpoint of further improving the emulsion stability, preferred as the plant protein are proteins derived from at least any among soybean, pea, fava bean, lentil, rice, barley, sorghum, potato, coconut, and pistachio, more preferred are proteins derived from at least any among soybean, pea, fava bean, lentil, rice, sorghum, and pistachio, and particularly preferred are proteins derived from at least any among lentil, rice, and pistachio.

In the agent for solubilization and emulsion stability improvement described above, the type, the use amount, etc. of the component to be used are as described in the section "1. Method for producing processed plant protein-containing composition".

2-4. Agent for solubilization and foamability improvement

The combination of the protein deamidase and the transglutaminase can improve the foamability in addition to the solubilization of a plant protein-containing liquid composition derived from a plant selected from the group consisting of pea, mung bean, chickpea, and almond. Accordingly, the present invention also provides an agent for solubilization and foamability improvement of a plant protein-containing liquid compositions derived from these specific plants, including a protein deamidase and a transglutaminase.

From the viewpoint of further improving the foamability, preferred as the plant protein are proteins derived from at least any of chickpea and almond, and particularly preferred is a protein derived from almond.

In the agent for solubilization and foamability improvement described above, the type, the use amount, etc. of the component to be used are as described in the section "1. Method for producing processed plant protein-containing composition".

2-5. Agent for solubilization and foam stability improvement

The combination of the protein deamidase and the transglutaminase can improve the foam stability in addition to the solubilization of a plant protein-containing liquid composition derived from a plant selected from the group consisting of soybean, fava bean, rice, and potato. Accordingly, the present invention also provides an agent for solubilization and foam stability improvement of a plant protein-containing liquid compositions derived from these specific plants, including a protein deamidase and a transglutaminase.

From the viewpoint of further improving the foam stability, preferred as the plant protein are proteins derived from at least any among soybean, fava bean, and potato, and particularly preferred is a protein derived from soybean.

In the agent for solubilization and foam stability improvement described above, the type, the use amount, etc. of the component to be used are as described in the section "1. Method for producing processed plant protein-containing composition".

2-6. Agent for solubilization and oil retentivity improvement

The combination of the protein deamidase and the transglutaminase can improve the oil retentivity in addition to the solubilization of a plant protein-containing liquid composition derived from a plant selected from the group consisting of fava bean, chickpea, lentil, rice, potato, cashew nut, peanut, pistachio, walnut, chia seed, and hemp seed. Accordingly, the present invention also provides an agent for solubilization and oil retentivity improvement of a plant protein-containing liquid compositions derived from these specific plants, including a protein deamidase and a transglutaminase.

From the viewpoint of further improving the oil retentivity, preferred as the plant protein are proteins derived from at least any among lentil, potato, cashew nut, pistachio, chia seed, and hemp seed, more preferred are proteins derived from at least any among potato, cashew nut, pistachio, and chia seed, and particularly preferred are proteins derived from at least any of potato and chia seed.

In the agent for solubilization and oil retentivity improvement described above, the type, the use amount, etc. of the component to be used are as described in the section "1. Method for producing processed plant protein-containing composition".

2-7. Agent for solubilization and smoothness improvement

The combination of the protein deamidase and the transglutaminase can improve the smoothness in addition to the solubilization of a plant protein-containing liquid composition derived from a plant selected from the group consisting of soybean, pea, mung bean, lupine bean, rice, and almond. Accordingly, the present invention also provides an agent for solubilization and smoothness improvement of a plant protein-containing liquid compositions derived from these specific plants, including a protein deamidase and a transglutaminase.

From the viewpoint of further improving the smoothness, preferred as the plant protein are proteins derived from at least any among soybean, pea, mung bean, lupine bean, and rice.

In the agent for solubilization and smoothness improvement described above, the type, the use amount, etc. of the component to be used are as described in the section "1. Method for producing processed plant protein-containing composition".

3. Solubilizer of plant protein-containing liquid composition to be treated with protein deamidase

A transglutaminase can solubilize the protein of the plant protein-containing liquid composition to be treated with a protein deamidase. Therefore, the present invention also provides a solubilizer of a plant protein-containing liquid composition to be treated with a protein deamidase, including a transglutaminase. The solubilizer of the plant protein-containing liquid composition to be treated with a protein deamidase may be in the form of an enzyme preparation including a transglutaminase.

Regarding the above solubilizer, the term "solubilization" means to impart a property of further increasing the amount of protein to be dissolved in water (that is, improvement of solubility) to a plant protein-containing liquid composition as compared with the case of being solubilized only by a protein deamidase. Specific modes of use of the solubilizer include all of a mode in which the plant protein-containing liquid composition is treated with a solubilizer and a protein deamidase simultaneously, a mode in which the plant protein-containing liquid composition is treated with a protein deamidase and then treated with a solubilizer, and a mode in which the plant protein-containing liquid composition is treated with a solubilizer and then treated with a protein deamidase.

From the viewpoint of further improving the solubility, preferred as the plant protein are proteins derived from at least any among soybean, pea, mung bean, fava bean, chickpea, lentil, lupine bean, rice, barley, oat, sorghum, rye, corn, potato, hazelnut, cashew nut, almond, coconut, peanut, pistachio, walnut, chia seed, and hemp seed, more preferred are proteins derived from at least any among barley, oat, sorghum, corn, potato, lupine bean, hazelnut, and hemp seed, still more preferred are proteins derived from at least any among barley, corn, potato, and hemp seed, further preferred are proteins derived from at least any of corn and hemp seed, and particularly preferred is a protein derived from corn.

In the above solubilizer, the type, the use amount, etc. of the component to be used are as described in the section "1. Method for producing processed plant protein-containing composition".

4. Dispersion stability improver of plant protein-containing liquid composition to be treated with protein deamidase

A transglutaminase can improve the dispersion stability of a plant protein-containing liquid composition to be treated with a protein deamidase. Therefore, the present invention also provides a dispersion stability improver of a plant protein-containing liquid composition to be treated with a protein deamidase, including a transglutaminase.

Regarding the above dispersion stability improver, the term "dispersion stability improvement" means to impart a property of further suppressing precipitation of an insoluble matter contained in a plant protein-containing liquid composition to the plant protein-containing liquid composition as compared with the case of improving the dispersion stability only by a protein deamidase. Specific modes of use of the dispersion stability improver include all of a mode in which the plant protein-containing liquid composition is treated using the dispersion stability improver and a protein deamidase simultaneously, a mode in which the plant protein-containing liquid composition is treated with a protein deamidase and then treated with the dispersion stability improver, and a mode in which the plant protein-containing liquid composition is treated with the dispersion stability improver and then treated with a protein deamidase.

From the viewpoint of further improving the dispersion stability, the plant protein is preferably an oat-derived protein.

In the above dispersion stability improver, the type, the use amount, etc. of the component to be used are as described in the section "1. Method for producing processed plant protein-containing composition".

5. Plant protein-containing food or drink

The plant protein-containing food or drink of the present invention includes a processed plant protein-containing composition to be obtained by the above "1. Method for producing processed plant protein-containing composition". The processed plant protein-containing composition is as described above in "1. Method for producing processed plant protein-containing composition".

A specific form of the plant protein-containing food or drink of the present invention can be selected from among any food or drink forms. When the plant protein-containing food or drink of the present invention is to be eaten in a fluid form like a drink, examples of the form include not only a liquid state (for example, a liquid state drink) but also a dry state (for example, a powder drink and a freeze-dried drink).

Specific examples of the plant protein-containing food or drink are not particularly limited as long as they are alternative foods or drinks provided by replacing an animal protein material with a plant protein material, and examples thereof include a plant alternative milk, an alternative yogurt, an alternative cheese, an alternative ice cream, an alternative cream, an alternative coffee whitener, an alternative meat (including fish meat), an alternative egg, and an alternative seafood paste product.

The plant protein-containing food or drink can be obtained using the processed plant protein-containing composition as received or through an optional preparing process. In such a preparing process, a preparing method according to the form of the plant protein-containing food or drink is selected, and specifically, seasoning, form adjustment, molding, heat-cooking, fermentation, freezing, or the like may be performed.

Examples

Hereinafter, the present invention will be specifically described with reference to Examples, but the present invention is not to be construed as being limited to the following Examples.

[Enzymes used]

In the following Examples, the following enzymes were used.

[Table 1]

| Enzymes used | Protein deamidase | Protein glutaminase derived from Chryseobacterium proteolyticum | Amano Enzyme Inc. |
|---|---|---|---|
| | Transglutaminase | Transglutaminase derived from Streptomyces mobaraensis | Amano Enzyme Inc. |
| | α-Amylase | α-Amylase derived from Bacillus amyloliquefaciens | Amano Enzyme Inc. |
| | β-Amylase | β-Amylase derived from Bacillus flexus | Amano Enzyme Inc. |

[Method of measuring enzyme activity]

(1) Method of measuring protein deamidase activity

To 1 mL of a 0.2 M phosphate buffer (pH 6.5) containing 30 mM Z-Gln-Gly, 0.1 mL of a sample solution containing a protein deamidase was added, the mixture was allowed to stand at 37°C for 10 minutes, and then 1 mL of a 0.4 M TCA solution was added to stop the reaction. To 1 mL of a 0.2 M phosphate buffer (pH 6.5) containing 30 mM Z-Gln-Gly, 1 mL of a 0.4 M TCA reagent was added, 0.1 mL of a sample solution containing a protein deamidase was further added, and the mixture was allowed to stand at 37°C for 10 minutes as a blank.

The solution obtained above was measured using Ammonia Test Wako (FUJIFILM Wako Pure Chemical Corporation) to determine the amount of ammonia generated in the reaction liquid. A calibration curve representing the relationship between the ammonia concentration and the absorbance (630 nm) was prepared using an ammonia standard solution (ammonium chloride), and from the calibration curve, the ammonia concentration in the reaction liquid was determined.

The amount of the enzyme that produces 1 μmol of ammonia per minute was defined as 1 unit (1 U), and the activity of the protein deamidase was calculated from the following formula. In the formula, the reaction liquid amount is 2.1, the enzyme solution amount is 0.1, and Df is a dilution rate of the enzyme solution. 17.03 is a molecular weight of ammonia.

Protein deamidase activity (U/mL) = ammonia concentration in reaction liquid (mg/L) $\times$ (1/17.03) $\times$ (reaction liquid amount/enzyme solution amount) $\times$ (1/10) $\times$ Df          [Equation 1]

(2) Method of measuring transglutaminase activity

The enzyme activity of the transglutaminase was measured by the method described below using benzyloxycarbonyl-L-glutaminylglycine and hydroxylamine as substrates.

A substrate solution was prepared by dissolving 2.42 g of 2-amino-2-hydroxymethyl-1.3-propanediol, 0.70 g of hydroxyammonium hydrochloride, 0.31 g of reduced glutathione, and 1.01 g of Z-Gln-Gly (benzyloxycarbonyl-L-glutaminylglycine) in distilled water to make a total amount of 100 mL (pH 6.0).

A coloring solution was prepared by mixing 30 mL of a 3 M hydrochloric acid solution, 30 mL of a 12% by weight trichloroacetic acid solution, and 30 mL of 5% by weight iron(III) chloride solution.

An enzyme was diluted with a 200 mM MES buffer (pH 6.0) to an appropriate concentration to prepare a sample solution.

100 μL of the substrate solution was added to 10 μL of the sample solution and mixed, and then the mixture was reacted at 37°C for 10 minutes. 100 μL of the coloring solution was added to stop the reaction and form a Fe complex, and then the absorbance at 525 nm was measured. As a control, a sample solution heat-inactivated in advance was used and reacted in the same manner, then the absorbance thereof was measured, and the absorbance difference from the non-inactivated sample solution was determined. Separately, a calibration curve was created using L-glutamic acid-γ-monohydroxamate instead of the sample solution, and the amount of hydroxamic acid generated from the absorbance difference was determined. As for the activity of the transglutaminase, the amount of the enzyme that produces 1 μmol of hydroxamic acid per minute was defined as 1 unit (1 U).

(3) Method of measuring α-amylase activity

A potato starch was used as a substrate, the potato starch was dried in advance at 105°C for 2 hours, 1.0 g of the dried product thereof was weighed, 20 mL of water was added, and 5 mL of a sodium hydroxide test solution (2 mol/L) was gradually added with stirring to form a paste. Next, the paste was heated in a boiling water bath for 3 minutes with stirring, and then 25 mL of water was added. After cooling, the mixture was neutralized by adding a hydrochloric acid test solution (2 mol/L) and a hydrochloric acid test solution (0.1 mol/L), 10 mL of a 1 mol/L acetic acid-sodium acetate buffer solution (pH 5.0) was added, and water was further added to make 100 mL, thereby affording a 1% potato starch substrate solution (0.1 mol/L acetic acid (pH 5.0)).

10 mL of the 1% potato starch substrate solution (0.1 mol/L acetic acid (pH 5.0)) was warmed at 37°C for 10 minutes, 1 mL of a sample solution containing α-amylase was then added, and the mixture was immediately shaken. This solution was left standing at 37°C for 10 minutes, then 1 mL of this solution was added to 10 mL of a 0.1 mol/L hydrochloric acid test solution, and the mixture was immediately shaken. Next, 0.5 mL of this solution was weighed, and 10 mL of a 0.0002 mol/L iodine test solution (Japanese Pharmacopoeia) was added thereto. The mixture was shaken, and then absorbance (AT) at a wavelength of 660 nm was measured using water as a control. Separately, a similar operation was performed except that 1 mL of water was added instead of the sample solution, and the absorbance (AB) was measured. The activity of α-amylase was calculated from the following formula where the amount of the enzyme that reduces the color of potato starch caused by iodine by 10% per minute was defined as 1 unit (1 U).

[Equation 2]

$$\alpha\text{-Amylase activity (U/g, U/mL)} = (AB - AT)/AB \times 1/W$$

AT: Absorbance of reaction solution
AB: Absorbance of blank liquid
W: Amount of sample (g or mL) in 1 mL of sample solution

(4) Method of measuring β-amylase activity

A potato starch was used as a substrate, the potato starch was dried in advance at 105°C for 2 hours, 1.0 g of the dried product thereof was weighed, 20 mL of water was added, and 5 mL of a sodium hydroxide test solution (2 mol/L) was gradually added with stirring to form a paste. Next, the paste was heated in a boiling water bath for 3 minutes with stirring, and then 25 mL of water was added. After cooling, the mixture was neutralized by adding a hydrochloric acid test solution (2 mol/L) and a hydrochloric acid test solution (0.1 mol/L), 10 mL of a 1 mol/L acetic acid-sodium acetate buffer solution (pH 5.0) was added, and water was further added to make 100 mL, thereby affording a substrate solution.

10 mL of the substrate solution was weighed and warmed at 37°C for 10 minutes, 1 mL of a sample solution was added and immediately shaken, the mixture was warmed at the same temperature for 10 minutes, 4 mL of a Fehling's test solution was added and shaken lightly, the mixture was heated in a boiling water bath for 15 minutes, and then cooled to 25°C or lower, and 2 mL of a potassium iodide solution (a solution prepared by dissolving 30 g of potassium iodide in 70 mL of water) and 2 mL of 3 mol/L sulfuric acid were added, thereby affording a test solution. The Fehling's test solution was prepared at the time of use by mixing a copper solution prepared by weighing 34.66 g of fine crystals of copper(II) sulfate pentahydrate and adding water thereto to make 500 mL, and an alkaline tartrate solution prepared by weighing 173 g of (+)-potassium sodium tartrate tetrahydrate and 50 g of sodium hydroxide and adding water thereto to make 500 mL, in a ratio of 1 volume of the copper solution to 1 volume of the alkaline tartrate solution. Separately, the same operation as in the preparation of the test solution was performed using 10 mL of water instead of the substrate solution to prepare a comparative solution. For the test solution and the comparative solution, liberated iodine was titrated with a 0.05 mol/L sodium thiosulfate solution. The end point was set to the time at which one or two drops of a soluble starch test solution were added when the titration was close to the end point, and the resulting blue color disappeared.

The amount of the enzyme that leads to an increase in reducing power corresponding to 1 mg of glucose per minute was defined as 1 unit (1 U), and the activity of the β-amylase was calculated by the following formula.

[Equation 3]

$$\beta\text{-Amylase activity (U/g, U/mL)} = \text{amount of glucose (mg)} \times 1/10 \times 1/M$$

$$\text{Amount of glucose (mg)} = (b - a) \times 1.6 \times f$$

a: Titration value (mL) of enzyme reaction solution
b: Titration value (mL) of blank liquid
1.6: 1 mL of 0.05 mol/L sodium thiosulfate solution corresponds to 1.6 mg of glucose amount
1/10: Unit conversion factor of reaction time (min)
M: Amount of sample (g or mL) in 1 mL of sample solution
f: Factor of 0.05 mol/L sodium thiosulfate solution (for titration)

[Plant protein material used]

In the following Examples, the following plant protein material was used.

· Oat flour: "Premium oat flour" (manufactured by Slow Food Co., Ltd.), protein content: 13.1% by weight, carbohydrate content: 67.7% by weight

[Test Example 1]

(1) Production of processed oat milk

18.1 g of the oat flour and 150 g of water were mixed and suspended in a 200 mL Erlenmeyer flask. This suspension was adjusted to pH 6.5, 10 U/g-starch α-amylase and 0.09 U/g-starch β-amylase were added thereto, and the mixture was allowed to react at 54°C for 1 hour. The protein deamidase (PG) and the transglutaminase (TG) having the activity ratios shown in Table 2-1 and Table 2-2 were added such that the total amount thereof was 0.063% by weight, and pH was adjusted to 8.0, then the mixture was allowed to react at 60°C for 1 hour. After the reaction, the enzymes were deactivated by heat treatment at 95°C for 5 minutes. The mixture was centrifuged at 1,000 rpm for 1 minute, and the supernatant was collected. The supernatant collected was adjusted to pH 6.0 (at 25°C), affording processed oat milk (Examples 1 to 3, Comparative Examples 2 and 3). Separately, a processed oat milk was obtained in the same manner as described above except that no enzymes were added (Comparative Example 1).

(2) Characteristic test of processed oat milk

The processed oat milks obtained were subjected to the following characteristic tests.

(2-1) Protein solubility (solubility)

· Method for preparing BCA solution

50 mL of BCA Protein Assay Reagent (Reagent A Pierce) and 1 mL of BCA Protein Assay Reagent (Reagent B Pierce) were well mixed to prepare a BCA solution.

· Method for measuring protein solubility

4 mL of the BCA solution was weighed, and allowed to stand at 37 ± 0.5°C for exactly 10 minutes. Then, 0.2 mL of a sample diluted solution (50-fold diluted processed oat milk) was added, and the mixture was immediately shaken. This solution was allowed to stand at 37 ± 0.5°C for exactly 30 minutes, and then cooled in running water (20 to 25°C). The absorbance (AT) of this solution at a wavelength of 562 nm was measured using water as a control. Separately, 4 mL of the BCA solution was allowed to stand at 37 ± 0.5°C for exactly 10 minutes, and then 0.2 mL of water was added and shaken, and thereafter, the absorbance (AB) of the mixture was measured by the same operation as described above.

The albumin concentration Tmg in the sample diluted solution was determined from the absorbance difference (AT - AB) and an albumin standard curve. The protein solubility (mg/g) in the processed oat milk was determined on the basis of the obtained albumin concentration Tmg. The results are shown in Table 2-1.

(2-2) Dispersion stability

The processed oat milk obtained was left at rest in a refrigerator for 24 hours. At that time, the height of the entire liquid and the height of the sedimentation layer were measured, and the dispersion stability was determined on the basis of the following equation. The higher the numerical value of the dispersion stability is, the higher the dispersion stability is indicated to be. The results are shown in Table 2-2.

Dispersion stability (%) = 100 × (height of sedimentation layer)/(height of entire liquid)          [Equation 4]

(3) Test results

[Table 2-1]

|  | PG addition activity (U/g-protein) | TG addition activity (U/g-protein) | Activity ratio of PG to TG (PG activity : TG activity) | Protein solubility (mg/g) |
|---|---|---|---|---|
| Comparative Example 1 | 0 | 0 | No enzymes | 11.2 |
| Comparative Example 2 | 0 | 45 | 0:1 | 10.9 |
| Comparative Example 3 | 22.5 | 0 | 1:0 | 12.5 |
| **Example 1** | **4.5** | **36** | **0.11:0.89** | **12.2** |
| **Example 2** | **11.25** | **22.5** | **0.33:0.67** | **12.8** |
| **Example 3** | **18** | **9** | **0.67:0.33** | **13.2** |

As shown in Table 2-1, although TG alone (Comparative Example 2) reduced the solubility of the protein, the combination of TG with PG (Examples 1 to 3) significantly enhanced the effect of improving the solubility of the protein as compared with the case of PG alone (Comparative Example 3). For example, in Example 1, although PG was used in an amount of only 1/5 (in addition activity) of the amount of PG used in Comparative Example 3, and TG, which lowers the protein solubility, was further used in an amount of about 9 times that of PG, a protein solubility equivalent to that in Comparative Example 3 was achieved, whereas in Examples 2 and 3, although only PG was used in an amount (in addition activity) smaller than the amount of PG used in Comparative Example 3, the protein solubility was improved as compared with Comparative Example 3.

[Table 2-2]

|  | PG addition activity (U/g-protein) | TG addition activity (U/g-protein) | Activity ratio of PG to TG (PG activity : TG activity) | Dispersion stability (%) |
|---|---|---|---|---|
| Comparative Example 1 | 0 | 0 | No enzymes | 86.7 |
| Comparative Example 2 | 0 | 45 | 0:1 | 90 |
| Comparative Example 3 | 22.5 | 0 | 1:0 | 91.7 |
| **Example 1** | **4.5** | **36** | **0.11:0.89** | **95** |
| **Example 2** | **11.25** | **22.5** | **0.33:0.67** | **93.3** |
| **Example 3** | **18** | **9** | **0.67:0.33** | **93.3** |

As shown in Table 2-2, although the total weight of PG and TG was the same in all of Comparative Examples 2 and 3 and Examples 1 to 3, the effect of improving the dispersion stability was observed when PG and TG were combined (Examples 1 to 3).

[Test Example 2]

(1) Test Method

Processed oat milks were prepared in the same manner as in Example 3 (PG activity : TG activity = 0.67:0.33) of Test Example 1, and the protein solubility and the dispersion stability were measured. The addition amount of each enzyme was as shown in Table 3. A relative value of protein solubility and a relative value of dispersion stability were derived where the protein solubility and the dispersion stability of Example 5 (retest of Example 3) were each 1. The results are shown in Table 3.

(2) Test results

[Table 3]

| | PG addition activity (U/g-protein) | TG addition activity (U/g'-protein) | Activity ratio of PG to TG (PG activity : TG activity) | Protein solubility (mg/g) Relative value | Dispersion stability (%) Relative value |
|---|---|---|---|---|---|
| Example 4 | 1.8 | 0.9 | 0.67:0.33 | 0.96 | 0.95 |
| Example 5 | 18 | 9 | 0.67:0.33 | 1.00 | 1.00 |
| Example 6 | 180 | 90 | 0.67:0.33 | 1.07 | 1.02 |

As shown in Table 3, in all of Examples 4 to 6, improvement in protein solubility and improvement in dispersion stability were observed.

[Test Example 3]

(1) Test Method

Processed oat milks were prepared in the same manner as in Comparative Example 1 (no enzymes), Comparative Example 3 (PG activity : TG activity = 1:0), or Example 3 (PG activity : TG activity = 0.67:0.33) of Test Example 1 except that the pH of the processed oat milk and the addition amount of each enzyme were changed as shown in Table 4, and the dispersion stability was evaluated. The results are shown in Table 4.

(2) Test results

[Table 4]

| | pH | PG addition activity (U/g-protein) | TG addition activity (U/g-protein) | Dispersion stability (%) |
|---|---|---|---|---|
| Comparative Example 4 | | 0 | | 55 |
| Comparative Example 5 | 5 | 18 | 0 | 53.3 |
| Comparative Example 6 | | 180 | | 65 |
| Example 7 | 5 | 18 | 9 | 65 |
| Example 8 | | 180 | 90 | 72 |

(continued)

|  | pH | PG addition activity (U/g-protein) | TG addition activity (U/g-protein) | Dispersion stability (%) |
|---|---|---|---|---|
| Comparative Example 7 | 7 | 0 | 0 | 65 |
| Comparative Example 8 | | 0.9 | | 75 |
| Comparative Example 9 | | 1.8 | | 75 |
| Comparative Example 10 | | 180 | | 91.7 |
| **Example 9** | 7 | **0.9** | **0.45** | **85** |
| **Example 10** | | **1.8** | **0.9** | **88.3** |
| **Example 11** | | **18** | **9** | **93.3** |
| **Example 12** | | **180** | **90** | **93.3** |

As shown in Table 4, in Example 7, in which the amount of PG added and the amount of TG added were only 18 U and 9 U, respectively, per 1 g of protein, the dispersion stability was equivalent to that in Comparative Example 6, in which the amount of PG added was as much as 180 U per 1 g of the protein; in Example 9, in which the amount of PG added and the amount of TG added are only 0.9 U and 0.45 U, respectively, per 1 g of the protein, the dispersion stability was improved as compared with Comparative Example 9, in which the amount of PG added is as much as 1.8 U per 1 g of the protein; in Example 11, in which the amount of PG added and the amount of TG added were only 18 U and 9 U, respectively, per 1 g of the protein, the dispersion stability was improved as compared with Comparative Example 10, in which the amount of PG added was as much as 180 U per 1 g of the protein; from these results, it was recognized that a remarkable effect of improving the dispersion stability was obtained at any pH by using PG and TG in combination.

[Test Example 4]

(1) Production of processed plant protein-containing composition

Plant protein-containing liquid compositions were prepared by suspending the plant protein materials shown in Table 5 (powder; product pulverized as necessary with a mill to have the same particle size) in water to a concentration of 10% by weight. When a wholemeal cereal flour was used as a plant protein material, the pH of an aqueous suspension of the wholemeal flour was adjusted to 6.5, 10 U/g-starch $\alpha$-amylase and 0.09 U/g-starch $\beta$-amylase were added thereto, and the mixture was subjected to pretreatment involving a reaction at 54°C for 1 hour to prepare a plant protein-containing liquid composition. A protein deamidase (PG) was added in an amount of 18 U per 1 g of the protein, and a transglutaminase (TG) was added in an amount of 9 U per 1 g of the protein, and the pH at 25°C was adjusted to 6 to 7, and then the mixture was allowed to react at 50°C overnight. After the reaction, the enzymes were deactivated by heat treatment at 100°C for 5 minutes, affording a processed plant protein-containing liquid composition. The resulting processed plant protein-containing liquid composition was freeze-dried to afford a processed plant protein-containing dry composition (Examples 13 to 34). Separately, processed plant protein-containing dry compositions (Comparative Examples 13 to 34 with sub-numbers) were obtained by the same operation except for lacking at least one of PG and TG.

[Table 5]

| Product name | Plant protein | Protein content (% by weight) | Manufacturer |
|---|---|---|---|
| SOYPRO | Soybean protein | 50% or more | J-OIL MILLS, INC. |
| NUTRALYS F85M | Pea protein | 85% or more | Roquette |
| MUNG BEAN PROTEIN | Mung bean protein | 80% or more | Organo Corporation |
| FAVA BEAN PROTEIN (FAVA) | Fava bean protein | 83.7% or more | Organo Corporation |
| CHICKPEA PROTEIN | Chickpea protein | 84% or more | Organo Corporation |
| LENTIL PROTEIN | Lentil protein | 50% or more | Organo Corporation |
| RICE PROTEIN | Rice protein | 85% or more | Bio Actives |

(continued)

| Product name | Plant protein | Protein content (% by weight) | Manufacturer |
|---|---|---|---|
| BARLEY WHOLEMEAL FLOUR | Barley protein | 6.7% | TOMIZAWA SHOUTEN |
| SORGHUM WHOLEMEAL FLOUR | Sorghum protein | 9.6% | TOMIZAWA SHOUTEN |
| RYE WHOLEMEAL FLOUR | Rye protein | 8.5% | TOMIZAWA SHOUTEN |
| Zein | Zein (corn protein) | 81% | FUJIFILM Wako Pure Chemical Corporation |
| TUBERMINE® FV | Potato protein | 80% or more | Roquette Japan K.K. |
| HAZELNUT POWDER | Hazelnut protein | 13% | GABAN |
| CASHEW NUT POWDER | Cashew nut protein | 20% | MINOYA |
| ALMOND PROTEIN | Almond protein | 85% or more | Bio Actives |
| COCONUT FLAKE | Coconut protein | 7% | ALISHAN |
| PEANUT PROTEIN | Peanut protein | 26.5% | MORIMOTO SHOUTEN |
| PISTACHIO POWDER | Pistachio protein | 17% | MINOYA |
| WALNUT PROTEIN | Walnut protein | 14.6% | TOMIZAWA SHOUTEN |
| CHIA SEED PROTEIN | Chia seed protein | 83% or more | Organo Corporation |
| HEMP PROTEIN | Hemp seed* protein | 85% or more | Bio Actives |
| Lupine bean | Lupine bean protein | 85% | Wide Open Agriculture |
| *Hemp seed is derived from industrial hemp free of THC. | | | |

(2) Characteristic test of plant protein-containing liquid composition by redispersion in water

The processed plant protein-containing dry compositions obtained were subjected to the following characteristic tests.

(2-1) Protein solubility (solubility)

The processed plant protein-containing dry composition obtained was redispersed in water to prepare a redispersion liquid containing 10% by weight of the dry composition, and the redispersion liquid was left at rest at room temperature for 30 minutes. After the leaving, centrifugation was performed at 15,500 $\times$ g for 5 minutes and the supernatant was collected. The protein solubility (mg/mL) of the supernatant was measured using the method described in section (2-1) of Test Example 1. In addition, where the protein solubility according to Comparative Example of preparation using neither PG nor TG ("no enzymes") was 1, the relative value of the protein solubility of each Example (relative value of protein solubility/to no enzymes) was also derived. The higher the relative value of the protein solubility is, the higher the solubilization effect (that is, the effect of improving the solubility) is indicated to be. The results are shown in Table 6-1 to Table 6-4.

(2-2) Emulsifiability

The processed plant protein-containing dry composition was redispersed in deionized water to prepare 30 mL of a redispersion liquid containing 1% by weight of the protein. 30 mL of the redispersion liquid and 10 mL of canola oil were mixed and homogenized at 10,000 rpm for 2 minutes to prepare an emulsified composition. 50 $\mu$L of the emulsified composition immediately after the preparation was added to 5 mL of a 0.1% by weight SDS solution. The turbidity (A0) of the mixture was measured at an absorbance of 500 nm, and the emulsifiability ($m^2$/g) was calculated using the following calculation formula. In addition, where the emulsifiability by Comparative Example of preparation using neither PG nor TG ("no enzymes") was 1, the relative value of the emulsifiability of each Example (relative value of emulsifiability/to no enzymes) was also derived. The higher the relative value of emulsifiability is, the higher the effect of improving the emulsifiability is indicated to be. The results are shown in Table 6-1 to Table 6-4.

[Equation 5]

$$\text{Emulsifiability (m}^2/\text{g)} = (2 \times 2.303 \times A0)/(0.25 \times \text{protein weight})$$

(2-3) Emulsion stability

After the preparation in the above (2-2), 50 μL of the emulsified composition allowed to stand for 10 minutes was added to 5 mL of a 0.1% by weight SDS solution. The turbidity (A10) of the mixture was measured at an absorbance of 500 nm. The emulsion stability (%) was calculated using the following calculation formula. In addition, a value (emulsion stability increment) obtained by subtracting the emulsion stability according to Comparative Examples of preparation using neither PG nor TG from the emulsion stability of each Example was also derived. The higher the emulsion stability increment is, the higher the effect of improving the emulsion stability is indicated to be. The results are shown in Table 6-1 to Table 6-4.

[Equation 6]

$$\text{Emulsion stability (\%)} = \{(A10 \times 10)/(A0 - A10)\} \times 100$$

(2-4) Foamability

The processed plant protein-containing dry composition was redispersed in deionized water to prepare 50 mL of a redispersion liquid containing a 0.5% by weight dry composition. The redispersion liquid was homogenized at 18,000 rpm for 30 minutes and immediately transferred to a 100 mL graduated cylinder. The volume VF0 of the redispersion liquid including foam was measured, and the foamability (%) was calculated using the following calculation formula. In addition, where the foamability according to Comparative Example of preparation using neither PG nor TG ("no enzymes") was 1, the relative value of the foamability of each Example (relative value of foamability/to no enzymes) was also derived. The higher the relative value of foamability is, the higher the effect of improving the foamability is indicated to be. The results are shown in Table 6-1 to Table 6-4.

[Equation 7]

$$\text{Foamability (\%)} = 100 \times (VF0 - 50)/50$$

(2-5) Foam stability

For the redispersion liquid obtained by transferring the redispersion liquid homogenized in the above (2-4) to a 100 mL graduated cylinder and then allowing it to stand for 30 minutes, the volume VF30 of the redispersion liquid including foam was similarly measured, and the foam stability (%) was calculated using the following calculation formula. In addition, a value (foam stability increment) obtained by subtracting the foam stability according to Comparative Examples of preparation using neither PG nor TG from the foam stability of each Example was also derived. The higher the foam stability increment is, the higher the effect of improving the foam stability is indicated to be. The results are shown in Table 6-1 to Table 6-4.

[Equation 8]

$$\text{Foam stability (\%)} = 100 \times (VF30/VF0)$$

(2-6) Oil retentivity

An oil suspension was prepared by suspending 0.1 g of the processed plant protein-containing dry composition in 1 g of canola oil and vortexing the mixture for 30 seconds to prepare an oil suspension. The oil suspension was left at rest for 30 minutes, then centrifuged at 2,000 × g for 10 minutes, and the supernatant was collected. The weight of the collected supernatant was measured, and the obtained measured value was subtracted from 1 g (the weight of the canola oil used for suspension) to calculate the weight of the oil absorbed by the protein. Furthermore, assuming that 1 g of the processed plant protein-containing dry composition was 100%, the percentage of the weight (g) of the oil absorbed per 1 g of the processed plant protein-containing dry composition was taken as the oil retentivity (%). In addition, where the oil retentivity according to Comparative Example of preparation using neither PG nor TG ("no enzymes") was 1, the relative value of the oil retentivity of each Example (relative value of oil retentivity/to no enzymes) was also derived. The higher the relative value of oil retentivity is, the higher the effect improving the oil retentivity is indicated to be. The results are shown in Table 6-1 to Table 6-4.

(2-7) Smoothness

The processed plant protein-containing dry composition obtained was redispersed in water to prepare a redispersion liquid containing 10% by weight of the dry composition. Three trained panelists contained the redispersion liquid in their mouths, confirmed the texture (touch on the tongue), and classified on the basis of the following criteria regarding the grittiness in touch on the tongue, and the degree of imparting smoothness in comparison to each of Comparative Example prepared in the same manner except for lacking TG (that is, prepared using PG alone) and Comparative Example prepared in the same manner except for lacking PG (that is, prepared using TG alone). Examples in which the smoothness was improved as compared with PG alone and the smoothness was also improved as compared with TG alone were evaluated to have the effect of improving the smoothness by the combination of PG and TG. The results are shown in Table 6-1 to Table 6-4.

Grittiness is felt. ... "Y"
No grittiness is felt. ... "N"
Smoothness is not improved as compared with PG alone or TG alone. ... "-"
Smoothness is improved as compared with PG alone or TG alone. ... "+"
The smoothness is remarkably improved as compared with PG alone or TG alone.... "++"

(3) Test results

[Table 6-1]

| | Plant protein | PG addition activity (U/g-protein) | TG addition activity (U/g-protein) | Protein solubility (mg/mL) | Relative value of protein solubility/to no enzymes | Emulsifiability (m2/g) | Relative value of emulsifiability/to no enzymes | Emulsion stability (%) | Emulsion stability increment | Foamability (%) | Relative value of foamability/to no enzymes | Foam stability (%) | Foam stability increment | Oil retentivity (%) | Relative value of oil retentivity/to no enzymes | Grittiness in touch on tongue | Smoothness/to TG alone | Smoothness/to PG alone |
|---|---|---|---|---|---|---|---|---|---|---|---|---|---|---|---|---|---|---|
| Example 13 | Soybean | 18 | 9 | 2.0 | 2.09 | 6.0 | 1.34 | 62.9 | 30.6 | 6.7 | 0.56 | 96.2 | 5.2 | 4.6 | 0.92 | N | ++ | + |
| Comparative Example 13-1 | | 18 | 0 | - | - | - | - | - | - | - | - | 94.0 | - | 4.8 | - | N | | |
| Comparative Example 13-2 | | 0 | 9 | 1.6 | - | 5.0 | - | 39.7 | - | 8.7 | - | 93.9 | - | 4.4 | - | Y | | |
| Comparative Example 13-3 | | 0 | 0 | 1.0 | 1 | 4.5 | 1 | 32.3 | - | 12.0 | 1- | 91.1 | - | 5.0 | 1 | Y | | |
| Example 14 | Pea | 18 | 9 | 11.9 | 1.47 | 54.8 | 1.08 | 52.2 | 27.8 | 27.3 | 1.05 | 96.9 | -1.0 | 4.0 | 0.85 | N | ++ | + |
| Comparative Example 14-1 | | 18 | 0 | - | - | - | - | - | - | - | - | - | - | 3.7 | - | N | | |
| Comparative Example 14-2 | | 0 | 9 | 8.7 | - | 50.9 | - | 41.2 | - | 13.3 | - | 97.7 | - | 3.2 | - | Y | | |
| Comparative Example 14-3 | | 0 | 0 | 8.1 | 1 | 50.6 | 1 | 24.4 | - | 26.0 | 1 | 97.9 | | 4.7 | 1 | Y | | |

EP 4 585 694 A1

| | Plant protein | PG addition activity (U/g-protein) | TG addition activity (U/g-protein) | Protein solubility (mg/mL) | Relative value of protein solubility/to no enzymes | Emulsifiability (m2/g) | Relative value of emulsifiability/to no enzymes | Emulsion stability (%) | Emulsion stability increment | Foamability (%) | Relative value of foamability/to no enzymes | Foam stability (%) | Foam stability increment | Oil retentivity (%) | Relative value of oil retentivity/to no enzymes | Grittiness in touch on tongue | Smoothness/to TG alone | Smoothness/to PG alone |
|---|---|---|---|---|---|---|---|---|---|---|---|---|---|---|---|---|---|---|
| Example 15 | Mung bean | 18 | 9 | 3.6 | 1.30 | 40.1 | 1.22 | 23.8 | -19.9 | 20.7 | 1.03 | 85.7 | -11.0 | 3.7 | 0.68 | N | ++ | + |
| Comparative Example 15-1 | | 18 | 0 | - | - | - | - | - | - | - | - | - | - | 3.3 | - | N | | |
| Comparative Example 15-2 | | 0 | 9 | 3.0 | - | - | - | 36.0 | | 9.3 | - | 98.8 | - | 3.7 | - | Y | | |
| Comparative Example 15-3 | | 0 | 0 | 2.8 | 1 | 32.8 | 1 | 43.7 | - | 20.0 | 1 | 96.7 | - | 5.4 | 1 | Y | | |
| Example 16 | Fava bean | 18 | 9 | 3.3 | 2.13 | 71.8 | 1.67 | 55.3 | 30.6 | 14.0 | 0.49 | 98.3 | 2.9 | 3.1 | 1.03 | N | + | - |
| Comparative Example 16-1 | | 18 | 0 | - | - | - | - | - | - | - | - | 97.2 | - | - | - | N | | |
| Comparative Example 16-2 | | 0 | 9 | 2.1 | - | 69.3 | - | 50.7 | - | 14.7 | - | 97.7 | - | 2.5 | - | Y | | |
| Comparative Example 16-3 | | 0 | 0 | 1.6 | 1 | 43.0 | 1 | 24.8 | - | 28.7 | 1 | 95.3 | - | 3.0 | 1 | Y | | |

(continued)

| Plant protein | PG addition activity (U/g-protein) | TG addition activity (U/g-protein) | Protein solubility (mg/mL) | Relative value of protein solubility/to no enzymes | Emulsifiability (m2/g) | Relative value of emulsifiability/to no enzymes | Emulsion stability (%) | Emulsion stability increment | Foamability (%) | Relative value of foamability/to no enzymes | Foam stability (%) | Foam stability increment | Oil retentivity (%) | Relative value of oil retentivity/to no enzymes | Grittiness in touch on tongue | Smoothness/to TG alone | Smoothness/to PG alone |
|---|---|---|---|---|---|---|---|---|---|---|---|---|---|---|---|---|---|
| Example 17 | 18 | 9 | 2.5 | 1.45 | 61.9 | 1.47 | 43.3 | -31.5 | 4.0 | 1.20 | 99.4 | -0.6 | 2.9 | 1.16 | N | + | - |
| Comparative Example 17-1 | 18 | 0 | - | - | - | - | - | - | - | - | - | - | - | - | N | | |
| Comparative Example 17-2 | 0 | 9 | 2.0 | - | 60.3 | - | 69.6 | - | 2.0 | - | 100.0 | - | 2.5 | - | Y | | |
| Comparative Example 17-3 | 0 | 0 | 1.8 | 1 | 42.0 | 1 | 74.8 | - | 3.3 | 1 | 100.0 | ----- | 2.5 | 1 | Y | | |
| Example 18 | 18 | 9 | 4.5 | 1.18 | 42.2 | 1.32 | 59.6 | 39.2 | 16.7 | 0.45 | 92.6 | -3.0 | 3.4 | 1.23 | N | + | - |
| Comparative Example 18-1 | 18 | 0 | - | - | - | - | - | - | - | - | - | - | 3.3 | - | N | | |
| Comparative Example 18-2 | 0 | 9 | 1.4 | - | 37.5 | - | 51.5 | - | 17.3 | - | 96.6 | - | 2.9 | - | Y | | |
| Comparative Example 18-3 | 0 | 0 | 3.8 | 1 | 32.0 | 1 | 20.4 | - | 36.7 | 1 | 95.6 | - | 2.8 | 1 | Y | | |

Plant protein: Example 17 and Comparative Examples 17-1 to 17-3 = Chickpea; Example 18 and Comparative Examples 18-1 to 18-3 = Lentil.

[Table 6-2]

| | Plant protein | PG addition activity (U/g-protein) | TG addition activity (U/g-protein) | Protein solubility (mg/mL) | Relative value of protein solubility/to no enzymes | Emulsifiability (m2/g) | Relative value of emulsifiability/to no enzymes | Emulsion stability (%) | Emulsion stability increment | Foamability (%) | Relative value of foamability/to no enzymes | Foam stability (%) | Foam stability increment | Oil retentivity (%) | Relative value of oil retentivity/to no enzymes | Grittiness in touch on tongue | Smoothness/to TG alone | Smoothness/to PG alone |
|---|---|---|---|---|---|---|---|---|---|---|---|---|---|---|---|---|---|---|
| Example 19 | Rice | 18 | 9 | 2.1 | 2.04 | 6.4 | 1.39 | 89.1 | 41.0 | 10.7 | 0.44 | 98.2 | 0.9 | 3.3 | 1.13 | N | ++ | + |
| Comparative Example 19-1 | | 18 | 0 | - | - | - | - | - | - | - | - | 97.7 | - | 3.2 | - | Y | | |
| Comparative Example 19-2 | | 0 | 9 | 1.1 | - | - | - | 71.7 | - | 15.3 | - | 97.7 | - | 3.0 | - | Y | | |
| Comparative Example 19-3 | | 0 | 0 | 1.0 | 1 | 4.6 | 1 | 48.1 | - | 24.0 | 1 | 97.3 | - | 2.9 | 1 | Y | | |
| Example 20 | Barley | 18 | 9 | 6.5 | 1.21 | 6.8 | 0.79 | 100.0 | 15.2 | - | - | - | - | 3.8 | 0.83 | N | + | - |
| Comparative Example 20-1 | | 18 | 0 | 5.8 | - | - | - | - | - | - | - | - | - | 3.8 | - | N | | |
| Comparative Example 20-2 | | 0 | 9 | 5.9 | - | 7.5 | - | 90.0 | - | - | - | - | - | 4.3 | - | Y | | |
| Comparative Example 20-3 | | 0 | 0 | 5.4 | 1 | 8.6 | 1 | 84.8 | - | - | - | - | - | 4.5 | 1 | Y | | |

| | Plant protein | PG addition activity (U/g-protein) | TG addition activity (U/g-protein) | Protein solubility (mg/mL) | Relative value of protein solubility/to no enzymes | Emulsifiability (m2/g) | Relative value of emulsifiability/to no enzymes | Emulsion stability (%) | Emulsion stability increment | Foamability (%) | Relative value of foamability/to no enzymes | Foam stability (%) | Foam stability increment | Oil retentivity (%) | Relative value of oil retentivity/to no enzymes | Grittiness in touch on tongue | Smoothness/to TG alone | Smoothness/to PG alone |
|---|---|---|---|---|---|---|---|---|---|---|---|---|---|---|---|---|---|---|
| Example 21 | Sorghum | 18 | 9 | 5.3 | 1.07 | 5.6 | 0.69 | 78.1 | 28.5 | - | - | - | - | 5.0 | 0.85 | N | + | - |
| Comparative Example 21-1 | | 18 | 0 | 5.1 | - | - | - | - | - | - | - | - | - | 4.7 | - | N | | |
| Comparative Example 21-2 | | 0 | 9 | 5.0 | | 7.7 | | 66.6 | | - | - | - | - | 5.5 | | Y | | |
| Comparative Example 21-3 | | 0 | 0 | 5.0 | 1 | 8.3 | 1 | 49.5 | | - | - | - | - | 5.8 | 1 | Y | | |
| Example 22 | Rye | 18 | 9 | 7.1 | 2.29 | 8.4 | 0.66 | 50.9 | -11.5 | - | - | - | - | 3.9 | 0.84 | Y | - | - |
| Comparative Example 22-1 | | 18 | 0 | - | - | - | - | - | - | - | - | - | - | 3.9 | | Y | | |
| Comparative Example 22-2 | | 0 | 9 | 3.6 | - | 8.6 | - | 67.0 | - | - | - | - | - | 4.5 | - | Y | | |
| Comparative Example 22-3 | | 0 | 0 | 3.1 | 1 | 12.7 | 1 | 62.3 | - | - | - | - | - | 4.7 | 1 | Y | | |

26

| | Plant protein | PG addition activity (U/g-protein) | TG addition activity (U/g-protein) | Protein solubility (mg/mL) | Relative value of protein solubility/to no enzymes | Emulsifiability (m2/g) | Relative value of emulsifiability/-to no enzymes | Emulsion stability (%) | Emulsion stability increment | Foamability (%) | Relative value of foamability/to no enzymes | Foam stability (%) | Foam stability increment | Oil retentivity (%) | Relative value of oil retentivity/to no enzymes | Grittiness in touch on tongue | Smoothness/to TG alone | Smoothness/to PG alone |
|---|---|---|---|---|---|---|---|---|---|---|---|---|---|---|---|---|---|---|
| **Example 23** | Corn | 18 | 9 | 1.9 | 2.48 | 33.7 | 3.33 | 36.1 | -49.3 | - | - | - | - | 2.6 | 0.76 | N | ++ | - |
| Comparative Example 23-1 | | 18 | 0 | 1.0 | - | - | - | - | - | - | - | - | - | 2.5 | - | N | | |
| Comparative Example 23-2 | | 0 | 9 | 1.2 | - | 9.2 | - | 79.9 | - | - | - | - | - | 3.7 | - | Y | | |
| Comparative Example 23-3 | | 0 | 0 | 0.8 | 1 | 10.1 | 1 | 95.5 | - | - | - | - | - | 3.4 | 1 | Y | | |
| **Example 24** | Potato | 18 | 9 | 1.6 | 3.08 | 5.8 | 0.84 | 54.2 | 13.1 | 14.0 | 0.49 | 98.3 | 2.9 | 3.9 | 1.40 | | | |
| Comparative Example 24-1 | | 18 | 0 | 1.4 | - | - | - | - | - | - | - | 97.2 | - | 3.3 | - | | | |
| Comparative Example 24-2 | | 0 | 9 | 0.8 | - | 7.2 | - | 41.3 | - | 14.7 | - | 97.7 | - | 3.3 | - | | | |
| Comparative Example 24-3 | | 0 | 0 | 0.5 | 1 | 7.0 | 1 | 41.1 | - | 28.7 | 1 | 95.3 | - | 2.8 | 1 | | | |

[Table 6-3]

| | Plant protein | PG addition activity (U/g-protein) | TG addition activity (U/g-protein) | Protein solubility (mg/mL) | Relative value of protein solubility/to no enzymes | Emulsifiability (m2/g) | Relative value of emulsifiability/-to no enzymes | Emulsion stability (%) | Emulsion stability increment | Foamatility (%) | Relative value of foamability/to no enzymes | Foam stability (%) | Foam stability increment | Oil retentivity (%) | Relative value of oil retentivity/to no enzymes | Grittiness in touch on tongue | Smoothness/to TG alone | Smoothness/to PG alone |
|---|---|---|---|---|---|---|---|---|---|---|---|---|---|---|---|---|---|---|
| **Example 25** | | 18 | 9 | 4.8 | 1.62 | 16.0 | 0.63 | 46.2 | 5.7 | - | - | - | - | 2.5 | 0.96 | | | |
| Comparative Example 25-1 | Hazelnut | 18 | 0 | 4.7 | - | - | - | - | - | - | - | - | - | 2.5 | - | | | |
| Comparative Example 25-2 | | 0 | 9 | 2.7 | - | 19.1 | - | 39.6 | | - | - | - | - | 2.3 | - | | | |
| Comparative Example 25-3 | | 0 | 0 | 3.0 | 1 | 25.4 | 1 | 40.5 | - | - | - | - | - | 2.6 | 1 | | | |
| **Example 26** | | 18 | 9 | 2.8 | 1.21 | **17.9** | 1.45 | 45.9 | -26.3 | - | - | - | - | 2.9 | 1.33 | | | |
| Comparative Example 26-1 | Cashew **nut** | 18 | 0 | - | - | - | - | - | - | - | - | - | - | 2.7 | - | | | |
| Comparative Example 26-2 | | 0 | 9 | 1.6 | - | - | - | 13.4 | - | - | - | - | - | 2.7 | - | | | |
| Comparative Example 26-3 | | 0 | 0 | 2.3 | 1 | 12.3 | 1 | 72.2 | - | - | - | - | - | 2.2 | 1 | | | |

(continued)

| | Plant protein | PG addition activity (U/g-protein) | TG addition activity (U/g-protein) | Protein solubility (mg/mL) | Relative value of protein solubility/to no enzymes | Emulsifiabilty (m2/g) | Relative value of emulsifiability/to no enzymes | Emulsion stability (%) | Emulsion stability increment | Foamability (%) | Relative value of foamability/to no enzymes | Foam stability (%) | Foam stability increment | Oil retentivity (%) | Relative value of oil retentivity/to no enzymes | Grittiness in touch on tongue | Smoothness/to TG alone | Smoothness/to PG alone |
|---|---|---|---|---|---|---|---|---|---|---|---|---|---|---|---|---|---|---|
| **Example 27** | Almond | 18 | 9 | 3.6 | 1.64 | 38.0 | 1.67 | 12.2 | 0.6 | 12.0 | 2.00 | 99.2 | -1.1 | 3.4 | 0.77 | N | + | + |
| Comparative Example 27-1 | | 18 | 0 | - | - | - | - | - | - | - | - | - | - | 3.6 | - | N | | |
| Comparative Example 27-2 | | 0 | 9 | 1.7 | - | - | - | - | - | 0.7 | - | 99.3 | - | 3.5 | - | N | | |
| Comparative Example 27-3 | | 0 | 0 | 2.2 | 1 | 22.8 | 1 | 11.6 | - | 6.0 | 1 | 99.4 | - | 4.4 | 1 | Y | | |
| **Example 28** | Coconut | 18 | 9 | 0.3 | 2.09 | 16.3 | 2.27 | 52.6 | 17.1 | - | - | - | - | 5.5 | 0.80 | | | |
| Comparative Example 28-1 | | 18 | 0 | - | - | - | - | - | - | - | - | - | - | 5.8 | | | | |
| Comparative Example 28-2 | | 0 | 9 | 0.2 | - | 15.1 | - | 48.0 | - | - | - | - | - | 6.0 | | | | |
| Comparative Example 28-3 | | 0 | 0 | 0.1 | 1 | 7.2 | 1 | 35.4 | - | - | - | - | - | 6.9 | 1 | | | |

| | Plant protein | PG addition activity (U/g-protein) | TG addition activity (U/g-protein) | Protein solubility (mg/mL) | Relative value of protein solubility/to no enzymes | Emulsifiability (m2/g) | Relative value of emulsifiability/-to no enzymes | Emulsion stability (%) | Emulsion stability increment | Foamability (%) | Relative value of foamability/to no enzymes | Foam stability (%) | Foam stability increment | Oil retentivity (%) | Relative value of oil retentivity/to no enzymes | Grittiness in touch on tongue | Smoothness/to TG alone | Smoothness/to PG alone |
|---|---|---|---|---|---|---|---|---|---|---|---|---|---|---|---|---|---|---|
| **Example 29** | Peanut | 18 | 9 | **0.4** | **3.12** | 8.6 | 0.76 | **100.0** | 2.4 | - | - | - | - | 3.1 | 1.09 | N | + | - |
| Comparative Example 29-1 | | 18 | 0 | - | - | - | - | - | - | - | - | - | - | - | - | N | | |
| Comparative Example 29-2 | | 0 | 9 | 0.2 | - | 7.5 | - | 93.3 | - | - | - | - | - | 2.4 | - | Y | | |
| Comparative Example 29-3 | | 0 | 0 | 0.1 | 1 | **11.4** | 1 | 97.6 | - | - | - | - | - | 2.9 | 1 | Y | | |
| **Example 30** | Pistachio | **18** | **9** | **6.1** | **1.61** | **15.7** | **1.19** | **100.0** | **44.2** | - | - | - | - | **3.4** | **1.35** | | | |
| Comparative Example 30-1 | | 18 | 0 | - | - | - | - | - | - | - | - | - | - | 2.7 | - | | | |
| Comparative Example 30-2 | | 0 | 9 | 3.2 | - | 15.0 | - | 50.1 | - | - | - | - | - | 2.9 | - | | | |
| Comparative Example 30-3 | | 0 | 0 | 3.8 | 1 | 13.2 | 1 | 55.8 | - | - | - | - | - | 2.5 | 1 | | | |

[Table 6-4]

| | Plant protein | PG addition activity (U/g-protein) | TG addition activity (U/g-protein) | Protein solubility (mg/mL) | Relative value of protein solubility/to no enzymes | Emulsifiability (m2/g) | Relative value of emulsifiability/to no enzymes | Emulsion stability (%) | Emulsion stability increment | Foamability (%) | Relative value of foamability to no enzymes | Foam stability (%) | Foam stability increment | oil retentivity (%) | Relative value of oil retentivity/to no enzymes | Grittiness in touch on tongue | Smoothness/to TG alone | Smoothness/to PG alone |
|---|---|---|---|---|---|---|---|---|---|---|---|---|---|---|---|---|---|---|
| **Example 31** | Walnut | **18** | 9 | **18.4** | 1.45 | 28.2 | 2.82 | 67.8 | -21.0 | - | - | - | - | 3.4 | 1.16 | Y | - | - |
| Comparative Example 31-1 | | 18 | 0 | - | - | - | - | - | - | - | - | - | - | - | - | Y | | |
| Comparative Example 31-2 | | 0 | 9 | 10.9 | - | 24.2 | - | 89.5 | - | - | - | - | - | 3.3 | - | Y | | |
| Comparative Example 31-3 | | 0 | 0 | 12.7 | 1 | 10.0 | 1 | 88.8 | - | - | - | - | - | 2.9 | 1 | Y | | |
| **Example 32** | Chia seed | **18** | 9 | 2.5 | 1.44 | 8.5 | 1.13 | 76.3 | -5.8 | - | - | - | - | 3.3 | 1.46 | N | + | - |
| Comparative Example 32-1 | | 18 | 0 | - | - | - | - | - | - | - | - | - | - | 3.0 | - | N | | |
| Comparative Example 32-2 | | 0 | 9 | 1.0 | - | 8.1 | - | 83.9 | - | - | - | - | - | 2.5 | - | N | | |
| Comparative Example 32-3 | | 0 | 0 | 1.7 | 1 | 7.5 | 1 | 82.0 | - | - | - | - | - | 2.3 | 1 | Y | | |

| | Plant protein | PG addition activity (U/g-protein) | TG addition activity (U/g-protein) | Protein solubility (mg/mL) | Relative value of protein solubility/to no enzymes | Emulsifiability (m2/g) | Relative value of emulsifiability/to no enzymes | Emulsion stability (%) | Emulsion stability increment | Foamability (%) | Relative value of foamability to no enzymes | Foam stability (%) | Foam stability increment | oil retentivity (%) | Relative value of oil retentivity to no enzymes | Grittiness in touch on tongue | Smoothness/to TG alone | Smoothness/to PG alone |
|---|---|---|---|---|---|---|---|---|---|---|---|---|---|---|---|---|---|---|
| **Example 33** | Hemp seed | **18** | 9 | 3.4 | 2.29 | 19.5 | 0.78 | **98.6** | 9.8 | - | - | - | - | 2.8 | 1.24 | N | ++ | - |
| Comparative Example 33-1 | | 18 | 0 | 2.7 | - | - | - | - | - | - | - | - | - | 2.6 | - | N | | |
| Comparative Example 33-2 | | 0 | 9 | 2.0 | - | 20.6 | - | - | - | - | - | - | - | 2.3 | - | Y | | |
| Comparative Example 33-3 | | 0 | 0 | 1.5 | 1 | 25.0 | 1 | 88.9 | - | - | - | - | - | 2.3 | 1 | Y | | |
| **Example 34** | Lupine bean | **18** | 9 | **17.3** | 7.10 | - | - | - | - | - | - | - | - | - | - | N | ++ | + |
| Comparative Example 34-1 | | 18 | 0 | 16.8 | - | - | - | - | - | - | - | - | - | - | - | N | | |
| Comparative Example 34-2 | | 0 | 9 | 2.2 | - | - | - | - | - | - | - | - | - | - | - | Y | | |
| Comparative Example 34-3 | | 0 | 0 | 2.4 | 1 | - | - | - | - | - | - | - | - | - | - | Y | | |

EP 4 585 694 A1

32

As shown in Tables 6-1 to 6-4, in all of Examples 13 to 34, the effect of improving the solubility was observed as a result of combining PG and TG.

In regard to the plant protein-containing liquid compositions derived from soybean, pea, mung bean, fava bean, chickpea, lentil, rice, corn, cashew nut, almond, coconut, pistachio, walnut, or chia seed, improvement in emulsifiability was also observed in addition to improvement in solubilization (Examples 13 to 19, 23, 26 to 28, and 30 to 32).

With regard to the plant protein-containing liquid compositions derived from soybean, pea, fava bean, lentil, rice, barley, sorghum, potato, hazelnut, almond, coconut, peanut, pistachio, or hemp seed, improvement in emulsion stability was also observed in addition to improvement in solubilization (Examples 13, 14, 16, 18 to 21, 24, 25, 27 to 30, and 33).

In regard to the plant protein-containing liquid compositions derived from pea, mung bean, chickpea, or almond, improvement in foamability was also observed in addition to improvement in solubilization (Examples 14, 15, 17, and 27).

As to the plant protein-containing liquid compositions derived from soybean, fava bean, rice, or potato, improvement in foam stability was also observed in addition to improvement in solubilization (Examples 13, 16, 19, and 24).

With regard to the plant protein-containing liquid compositions derived from fava bean, chickpea, lentil, rice, potato, cashew nut, peanut, pistachio, walnut, chia seed, or hemp seed, improvement in oil retentivity was also observed in addition to improvement in solubilization (Examples 16 to 19, 24, 26, 29, and 30 to 33).

As to the plant protein-containing liquid compositions derived from soybean, pea, mung bean, lupine bean, rice, or almond, improvement in smoothness was also observed in addition to improvement in solubilization (Examples 13 to 15, 19, 27, and 34).

## Claims

1. A method for producing a processed plant protein-containing composition, comprising a step of treating a plant protein-containing liquid composition with a protein deamidase and a transglutaminase to obtain a processed plant protein-containing liquid composition.

2. The method for producing a processed plant protein-containing composition according to claim 1, further comprising a step of drying the processed plant protein-containing liquid composition to obtain a processed plant protein-containing dry composition for redispersion in a liquid.

3. The method for producing a processed plant protein-containing composition according to claim 1, wherein the plant protein is a plant-derived protein selected from the group consisting of soybean, pea, mung bean, fava bean, chickpea, lentil, lupine bean, rice, barley, oat, sorghum, rye, corn, potato, hazelnut, cashew nut, almond, coconut, peanut, pistachio, walnut, chia seed, and hemp seed.

4. The method for producing a processed plant protein-containing composition according to claim 1, wherein the protein deamidase and the transglutaminase are allowed to act simultaneously.

5. The method for producing a processed plant protein-containing composition according to claim 1, wherein an activity ratio of the protein deamidase to the transglutaminase is 0.05:0.95 to 0.9:0.1.

6. A solubilizer for a plant protein-containing liquid composition, comprising a protein deamidase and a transglutaminase.

7. The solubilizer according to claim 6, wherein an activity ratio of the protein deamidase to the transglutaminase is 0.05:0.95 to 0.9:0.1.

8. The solubilizer according to claim 6, which is used for improving dispersion stability of an oat-derived plant protein-containing liquid composition.

9. The solubilizer according to claim 8, wherein an activity ratio of the protein deamidase to the transglutaminase is 0.05:0.95 to 0.9:0.1.

10. The solubilizer according to claim 6 or 7, which is used for improving emulsifiability of a plant protein-containing liquid composition derived from a plant selected from the group consisting of soybean, pea, mung bean, fava bean, chickpea, lentil, rice, corn, cashew nut, almond, coconut, pistachio, walnut, and chia seed.

11. The solubilizer according to claim 6 or 7, which is used for improving emulsion stability of a plant protein-containing liquid composition derived from a plant selected from the group consisting of soybean, pea, fava bean, lentil, rice, barley, sorghum, potato, hazelnut, almond, coconut, peanut, pistachio, and hemp seed.

12. The solubilizer according to claim 6 or 7, which is used for improving foamability of a plant protein-containing liquid composition derived from a plant selected from the group consisting of pea, mung bean, chickpea, and almond.

13. The solubilizer according to claim 6 or 7, which is used for improving foam stability of a plant protein-containing liquid composition derived from a plant selected from the group consisting of soybean, fava bean, rice, and potato.

14. The solubilizer according to claim 6 or 7, which is used for improving oil retentivity of a plant protein-containing liquid composition derived from a plant selected from the group consisting of fava bean, chickpea, lentil, rice, potato, cashew nut, peanut, pistachio, walnut, chia seed, and hemp seed.

15. The solubilizer according to claim 6 or 7, which is used for improving smoothness of a plant protein-containing liquid composition derived from a plant selected from the group consisting of soybean, pea, mung bean, lupine bean, rice, and almond.

16. A plant protein-containing food or drink comprising a processed plant protein-containing composition obtained by the production method according to claim 1.

# INTERNATIONAL SEARCH REPORT

| | International application No. |
|---|---|
| | **PCT/JP2023/033068** |

| A. | CLASSIFICATION OF SUBJECT MATTER |
|---|---|

*C12P 21/00*(2006.01)i; *A23L 33/105*(2016.01)i; *C12N 9/10*(2006.01)i; *C12N 9/78*(2006.01)i; *C12P 1/00*(2006.01)i
FI:  C12P21/00 Z; C12N9/78; C12N9/10; A23L33/105; C12P1/00 A

According to International Patent Classification (IPC) or to both national classification and IPC

| B. | FIELDS SEARCHED |
|---|---|

Minimum documentation searched (classification system followed by classification symbols)

C12P21/00; A23L33/105; C12N9/10; C12N9/78; C12P1/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | WO 2009/113628 A1 (AJINOMOTO CO., INC.) 17 September 2009 (2009-09-17) claims 1-7, paragraphs [0002], [0003], [0009], [0011], [0025] | 1, 3-5, 16 |
| Y | | 1-16 |
| X | PORI, Pinja, et al. Enzymatic modification of oat protein concentrate with trans- and protein-glutaminase for increased fibrous structure formation during high-moisture extrusion processing. LWT-Food Science and Technology, December 2021,vol. 156, article no. 113035, Available online on 29 December 2021 abstract, left-hand column in p. 2, 2.2. Enzymatic modification of oat protein concentrate | 1, 3-5, 16 |
| Y | | 1-16 |
| X | WO 2021/259940 A2 (SOCIETE DES PRODUITS NESTLE S. A.) 30 December 2021 (2021-12-30) lines 18-32 in p. 4, lines 17-20 in p. 12, example 4 | 1, 3, 4, 16 |
| Y | | 1-16 |
| Y | WO 2022/102723 A1 (AMANO ENZYME INC.) 19 May 2022 (2022-05-19) claims 1-9, paragraph [0064] | 1-16 |

✓ Further documents are listed in the continuation of Box C.   ✓ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **26 September 2023** | **10 October 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

## INTERNATIONAL SEARCH REPORT

| International application No. |
| --- |
| **PCT/JP2023/033068** |

### C. DOCUMENTS CONSIDERED TO BE RELEVANT

| Category* | Citation of document, with indication, where appropriate, of the relevant passages | Relevant to claim No. |
| --- | --- | --- |
| Y | WO 2021/251344 A1 (AMANO ENZYME INC.) 16 December 2021 (2021-12-16) claims 1-10, paragraph [0008] | 1-16 |
| Y | WO 2022/019198 A1 (JTEKT CORP.) 27 January 2022 (2022-01-27) paragraph [0009] | 1-16 |
| A | WO 2022/039281 A1 (AJINOMOTO CO., INC.) 24 February 2022 (2022-02-24) claim 17 | 1-16 |
| A | WO 2020/171106 A1 (AMANO ENZYME INC.) 27 August 2020 (2020-08-27) claims | 1-16 |
| A | WO 2022/014542 A1 (AMANO ENZYME INC.) 20 January 2022 (2022-01-20) claims | 1-16 |
| A | WO 2022/092241 A1 (AMANO ENZYME INC.) 05 May 2022 (2022-05-05) claims | 1-16 |
| A | JP 2019-510514 A (OATLY AKTIEBOLAG) 18 April 2019 (2019-04-18) claims | 1-16 |
| A | WO 2022/063833 A1 (SOCIETE DES PRODUITS NESTLE S. A.) 31 March 2022 (2022-03-31) claims | 1-16 |
| A | WO 2022/165095 A1 (KANSAS STATE UNIVERSITY RESEARCH FOUNDATION) 04 August 2022 (2022-08-04) claims | 1-16 |

Form PCT/ISA/210 (second sheet) (January 2015)

## INTERNATIONAL SEARCH REPORT
### Information on patent family members

| | International application No. |
|---|---|
| | **PCT/JP2023/033068** |

| Patent document cited in search report | Publication date (day/month/year) | Patent family member(s) | Publication date (day/month/year) |
|---|---|---|---|
| WO 2009/113628 A1 | 17 September 2009 | US 2011/0064847 A1<br>claims, paragraphs [0005], [0006], [0014], [0027], [0051], claims 1-16<br>EP 2267146 A1<br>CN 102016057 A<br>KR 10-2010-0127824 A | |
| WO 2021/259940 A2 | 30 December 2021 | EP 4171245 A2<br>CN 115942878 A | |
| WO 2022/102723 A1 | 19 May 2022 | EP 4245149 A1<br>claims 1-9, paragraph [0073] | |
| WO 2021/251344 A1 | 16 December 2021 | EP 4162806 A1<br>paragraph [0008], claims 1-10<br>CN 115867144 A | |
| WO 2022/019198 A1 | 27 January 2022 | EP 4186965 A1<br>paragraph [0013]<br>CN 116134117 A | |
| WO 2022/039281 A1 | 24 February 2022 | US 2022/0053786 A1<br>CN 116056581 A | |
| WO 2020/171106 A1 | 27 August 2020 | US 2022/0151254 A1<br>claims<br>EP 3928627 A1<br>CN 113825403 A<br>KR 10-2021-0129112 A | |
| WO 2022/014542 A1 | 20 January 2022 | EP 4180528 A1<br>claims<br>CN 115715156 A | |
| WO 2022/092241 A1 | 05 May 2022 | EP 4238421 A1<br>claims<br>CN 114521592 A<br>claims | |
| JP 2019-510514 A | 18 April 2019 | WO 2017/171601 A1<br>claims<br>EP 3435781 A1<br>CN 109068684 A<br>KR 10-2019-0003540 A | |
| WO 2022/063833 A1 | 31 March 2022 | AU 2021350081 A1<br>claims<br>BR 112023004624 A2 | |
| WO 2022/165095 A1 | 04 August 2022 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6451369 B
- WO 2022071418 A
- JP 2002369653 A
- JP 2000050887 A
- JP 2001218590 A
- WO 2006075772 A1
- WO 2015133590 A

- JP S6427471 A
- JP 2020195397 A
- JP H0150382 B
- JP H06113844 A
- JP H5199883 A
- JP 2004097099 A
- WO 2009101762 A